# EUROPÄISCHE PATENTSCHRIFT

(11) **EP 1 124 797 B1**
(45) Veröffentlichungstag und Bekanntmachung des Hinweises auf die Patenterteilung: **03.03.2004**
(21) Anmeldenummer: 99971799.4
(22) Anmeldetag: 21.10.1999
(51) Int. Cl.: C07C 311/19, C07D 295/12, C07D 209/08, C07D 209/30, A61K 31/197, A61K 31/5375

(54) **N-ARYLSULFONYL-AMINOSÄURE-OMEGA-AMIDE**
N-ARYLSULFONYL AMINO ACID OMEGA AMIDES
OMEGA-AMIDES DE N-ARYLSULFONYLE-AMINOACIDE

(30) Priorität: 06.11.1998 DE 19851184
(43) Veröffentlichungstag der Anmeldung: 22.08.2001
(73) Patentinhaber: Aventis Pharma Deutschland GmbH, 65929 Frankfurt am Main (DE)
(72) Erfinder: SCHWAB, Wilfried, D-65207 Wiesbaden-Naurod (DE); THORWART, Werner, D-65239 Hochheim (DE); SCHUDOK, Manfred, D-65817 Eppstein (DE); HAASE, Burkhard, D-65719 Hofheim (DE)
(86) Internationale Anmeldenummer: PCT/EP1999/007979
(87) Internationale Veröffentlichungsnummer: WO 2000/027808

(56) Entgegenhaltungen:
- EP-A- 0 877 018
- WO-A-97/44315

## Beschreibung

Die Erfindung betrifft neue N-Aryl-sulfonyl-aminosäure-omega-amide, Verfahren zu ihrer Herstellung und Verwendung derselben als Arzneimittel.

In den Anmeldungen EP 0 606 046, WO 95/35276 und WO 96/27583 werden Arylsulfonaminohydroxamsäuren und deren Wirkung als Matrix-Metalloproteinase-Inhibitoren beschrieben. Spezielle Arylsulfonaminocarbonsäuren dienen als Zwischenprodukte zur Darstellung von Thrombin-Inhibitoren (EP 0 468 231) und Aldose-Reduktase-Inhibitoren (EP 0 305 947). In der Anmeldung EP 0 757 037 wird auch die Wirkung von Sulfonylaminosäure-Derivate als Metalloproteinase-Inhibitoren beschrieben.

In dem Bestreben wirksame Verbindungen zur Behandlung von Bindegewebserkrankungen zu finden, wurde nun gefunden, daß die erfindungsgemäßen Sulfonylaminocarbonsäuren starke Inhibitoren der Matrix-Metalloproteinasen sind. Dabei wird auf die Hemmung von Stromelysin (MMP-3) und der Neutrophilen Kollagenase (MMP-8) besonderer Wert gelegt, da beide Enzyme beim Abbau der Proteoglykane, als wichtige Bestandteile des Knorpelgewebes, maßgeblich beteiligt sind (A. J. Fosang et al. J. Clin. Invest. 98 (1996) 2292-2299). Ebenfalls zur Proteinfamilie der Matrix Metalloproteinasen gehören solche Enzyme, die am konstitutiven Ab- und Aufbau der Matrixbestandteile beteiligt sind. Beispielsweise kommt der MMP-1 (Kollagenase -1) eine wichtige vitale Funktion zu, weil sie am natürlichen Kollagenabbau beteiligt ist, insbesondere auch dort, wo morphogenetische Veränderungen stattfinden. Medizinische Wirkstoffe, welche zwar MMP-3 und MMP-8 zu hemmen vermögen, dabei aber MMP-1 weitgehend unbeinflußt lassen, sind also bevorzugt. Es kann sogar ein solcher Wirkstoff in Hinblick auf die Heilung des menschlichen oder tierischen Körpers besonders bevorzugt sein, der bei insgesamt nur moderater Inhibition von MMP-3 und -8 keinen oder einen schwächeren Effekt auf die MMP-1 zeigt.

Die Erfindung betrifft daher die Verbindung der Formel I 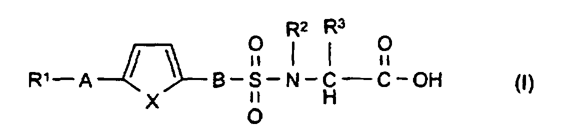 und/oder eine stereoisomere Form der Verbindung der Formel I und/oder ein physiologisch verträgliches Salz der Verbindung der Formel I, wobei
R¹ für
   1. Phenyl,
   2. Phenyl, welches ein- oder zweifach substituiert ist durch
      2.1. (C₁-C₆)-Alkyl, gerade, cyclisch oder verzweigt,
      2.2. Hydroxy,
      2.3. (C₁-C₆)-Alkyl-C(O)-O-,
      2.4. (C₁-C₆)-Alkyl-O-,
      2.5. (C₁-C₆)-Alkyl-O-(C₁-C₄)-Alkyl-O-,
      2.6. Halogen,
      2.7. -CF₃,
      2.8. -CN,
      2.9. -NO₂,
      2.10. HO-C(O)-,
      2.11. (C₁-C₆)-Alkyl-O-C(O)-,
      2.12. Methylendioxo,
      2.13. R⁴-(R⁵)N-C(O)-,
      2.14. R⁴-(R⁵)N-, oder
      2.15. einen Heterocyclus aus der nachfolgenden Gruppe Isoxazolidin, Morpholin, Isothiazolidin, Thiomorpholin, Pyrazolidin, Imidazolidin, Piperazin, Azetidin, Pyrrol, Pyrrolin, Pyrrolidin, Pyridin, Azepin, Piperidin, Oxazol, Isoxazol, Imidazol, Pyrazol, Thiazol, Isothiazol, Diazepin, Thiomorpholin, Pyrimidin und Pyrazin, der unsubstituiert oder wie unter 2.1 bis 2.14 beschrieben substituiert ist, oder
   3. einen Heteroaromaten aus der nachfolgenden Gruppe 3.1. bis 3.15., der unsubstituiert oder wie unter 2.1 bis 2.14 beschrieben substituiert ist,
      3.1. Pyrrol,
      3.2. Pyrazol,
      3.3. Imidazol,
      3.4. Triazol,
      3.5. Thiophen,
      3.6. Thiazol,
      3.7. Oxazol,
      3.8. Isoxazol,
      3.9. Pyridin,
      3.10. Pyrimidin,
      3.11. Indol,
      3.12. Benzothiophen,
      3.13. Benzimidazol,
      3.14. Benzoxazol oder
      3.15. Benzothiazol steht,
R², R⁴ und R⁵ gleich oder verschieden sind und für
   1. Wasserstoffatom,
   2. (C₁-C₆)-Alkyl-,
   3. HO-C(O)-(C₁-C₆)-Alkyl-,
   4. Phenyl-(CH₂)ₙ-, worin Phenyl unsubstituiert oder ein- oder zweifach wie unter 2.1. bis 2.14. beschrieben substituiert ist und n die ganze Zahl Null, 1 oder 2 darstellt, oder
   5. Picolyl stehen oder
   6. R⁴ und R⁵ zusammen mit der ringständigen Aminogruppe einen 4- bis 7-gliedrigen Ring bilden, worin gegebenenfalls eines der Kohlenstoffatome durch -O-, -S- oder -NH- ersetzt ist,
R³ für
   1. -(C₁-C₄)-Alkyl-C(O)-N(R⁶)-R⁷, worin R⁶ und R⁷ zusammen mit dem Stickstoff, an den sie gebunden sind einen Rest der Teilformel IIa, IIb oder IIe bilden, 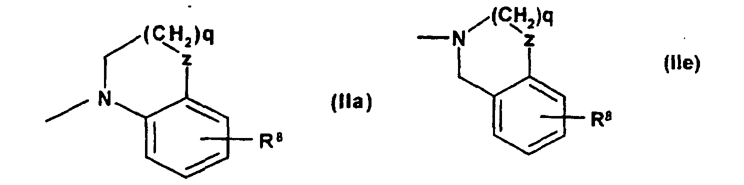 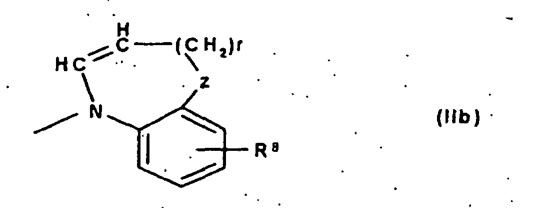 wobei in den Teilformeln IIa, IIb und IIe
      q die ganze Zahl Null, 1 oder 2 bedeutet und
      r die ganze Zahl Null oder 1 bedeutet,

      - Z: ein Kohlenstoff-, Stickstoff-, Sauerstoff- oder Schwefelatom oder eine kovalente Bindung bedeutet, und
      - R⁸: Wasserstoffatom bedeutet oder die für R¹ oben unter 2.1 bis 2.14 beschriebene Bedeutung hat,
   2. -(C₁-C₄)-Alkyl-C(O)-Y,
      worin Y einen Rest der Teifformel IIc oder IId bedeutet, 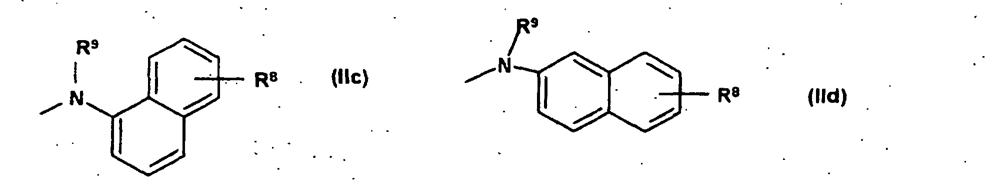 wobei in den Teilformeln IIc und IId
      - R⁸: Wasserstoffatom bedeutet oder die für R¹ oben unter 2.1 bis 2.14 beschriebene Bedeutung hat und
      - R⁹: für
      2.1 Wasserstoffatom
      2.2 (C₁-C₆)-Alkyl-,
      2.3 HO-C(O)-(C₁-C₆)-Alkyl-,
      2.4 Phenyl-(CH₂)ₙ-, worin Phenyl unsubstituiert oder ein- oder zweifach wie für R¹ unter 2.1 bis 2.14 beschrieben substituiert ist und n die ganze Zahl Null, 1 oder 2 darstellt, oder
      2.5 Picolyl steht, oder
   3. -(C₁-C₄)-Alkyl-C(O)-N(R⁹)-(CH₂)₀-N(R⁴)-R⁵, wobei
      - R⁹: die oben genannte Bedeutung hat,
      - o: die ganze Zahl 2, 3, 4 oder 5 bedeutet und
      - R⁴ und R⁵: zusammen mit der ringständigen Aminogruppe einen 4-bis 7-gliedrigen Ring bilden aus der Gruppe Isoxazolidin, Morpholin, Isothiazolidin, Thiomorpholin, Pyrazolidin, Imidazolidin, Piperazin, Azetidin, Pyrrolin, Pyrrolidin, Azepin, Piperidin oder Diazepin, steht,
A für
   a) eine kovalente Bindung,
   b) -O-,
   c) -CH=CH- oder
   d) -C≡C- steht,
B für
   a) -(CH₂)ₘ-, worin m die ganze Zahl Null, 1, 2, 3, 4, 5 oder 6 ist,
   b) -O-(CH₂)ₚ, worin p eine ganze Zahl von 1 bis 5 bedeutet, oder
   c) -CH=CH- steht und
X für -CH=CH-, Sauerstoffatom oder Schwefelatom steht,
mit Ausnahme der Verbindungen 2-(Biphenyl-4-sulfonylamino)-5-(2,3-dihydroihdol-1-yl)-5-oxo-pentancarbonsäure und 5-(2,3-Dihydroindol-1-yl)-5-oxo-2-(4'-pyrrolidin-1-yl-biphenyl-4-sulfonylamino)-pentancarbonsäure und mit Ausnahme des Falles, dass in der Teilformel IIb r die ganze Zahl Null und z eine kovalente Bindung darstellen.

Bevorzugt ist eine Verbindung der Formel I, wobei
R¹ für
   1. Phenyl oder
   2. Phenyl, welches einfach substituiert ist durch
      2.1. (C₁-C₆)-Alkyl-, worin Alkyl gerade, cyclisch oder verzweigt ist,
      2.2. -OH,
      2.3. (C₁-C₆)-Alkyl-C(O)-O-,
      2.4. (C₁-C₆)-Alkyl-O-,
      2.5. (C₁-C₆)-Alkyl-O-(C₁-C₄)-Alkyl-O-,
      2.6. Halogen,
      2.7. -CF₃ oder
      2.8. R⁴-(R⁵)N- steht,
R², R⁴ und R⁵ gleich oder verschieden sind und für
   1. Wasserstoffatom,
   2. (C₁-C₆)-Alkyl- stehen oder
   3. R⁴ und R⁵ zusammen mit der ringständigen Aminogruppe einen 4- bis 7-gliedrigen Ring bilden, worin gegebenenfalls eines der Kohlenstoffatome durch -O-, -S- oder -NH- ersetzt ist,
R³ für
   1. -(C₁-C₄)-Alkyl-C(O)-N(R⁶)-R⁷, worin R⁶ und R⁷ zusammen mit dem Stickstoff, an den sie gebunden sind einen Rest der Teilformel IIa bilden,
      wobei in der Teilformel IIa
      - q: die ganze Zahl Null oder 1 bedeutet,
      - Z: ein Kohlenstoff-, Stickstoff-, Sauerstoff- oder Schwefelatom bedeutet, und
      - R⁸: Wasserstoffatom bedeutet oder die für R¹ oben unter 2.1 bis 2.8 beschriebene Bedeutung hat,
   2. -(C₁-C₄)-Alkyl-C(O)-Y, worin
      - Y: einen Rest der Teilformel IIc oder IId bedeutet
      wobei in den Teilformeln IIc und IId
      R⁸ Wasserstoffatom bedeutet oder die für R¹ oben unter 2.1 bis 2.8 beschriebene Bedeutung hat und
      R⁹ für Wasserstoffatom steht, oder
   3. -(C₁-C₄)-Alkyl-C(O)-N(R⁹)-(CH₂)₀-N(R⁴)-R⁵, wobei
      - R⁹: für Wasserstoffatom steht,
      - o: die ganze Zahl 2 oder 3 bedeutet und
      - R⁴ und R⁵: gleich oder verschieden sind und für
      1. Wasserstoffatom,
      2. (C₁-C₆)-Alkyl- stehen oder
      3. R⁴ und R⁵ zusammen mit der ringständigen Aminogruppe einen 4-bis 7-gliedrigen Ring bilden aus der Gruppe Isoxazolidin, Morpholin, Isothiazolidin, Thiomorpholin, Pyrazolidin, Imidazolidin, Piperazin, Azetidin, Pyrrolin, Pyrrolidin, Azepin, Piperidin oder Diazepin, steht,
A für eine kovalente Bindung oder -O- steht,
B für
   a) -(CH₂)ₘ-, worin m die ganze Zahl Null, 1 oder 2 bedeutet, oder
   b) -O-(CH₂)ₚ, worin p die ganze Zahl 1 oder 2 bedeutet, steht und
X für -CH=CH- steht.

Bevorzugt ist ferner eine Verbindung der Formel I, wobei
R¹ für
   1. Phenyl oder
   2. Phenyl, welches einfach substituiert ist durch
      2.1. Chlor,
      2.2. Brom,
      2.3. Fluor,
      2.4. Pyrrolidin oder
      2.5. Morpholin steht,
R² für Wasserstoffatom steht,
R³ für
   1. -(C₁-C₄)-Alkyl-C(O)-N(R⁶)-R⁷, worin R⁶ und R⁷ zusammen mit dem Stickstoff, an den sie gebunden sind einen Rest der Teilformel IIa bilden, wobei in der Teilformel IIa
      - q: die ganze Zahl Null oder 1 bedeutet,
      - Z: ein Kohlenstoffatom bedeutet, und
      - R⁸: Wasserstoffatom, Chlor, Brom oder Fluor bedeutet,
   2. -(C₁-C₄)-Alkyl-C(O)-Y, worin
      - Y: einen Rest der Teilformel IIc oder IId bedeutet
      wobei in den Teilformeln IIc und IId
      R⁸ und R⁹ jeweils Wasserstoffatom bedeuten, oder
   3. -(C₁-C₄)-Alkyl-C(O)-N(R⁹)-(CH₂)₀-N(R⁴)-R⁵, wobei
      - R⁹: für Wasserstoffatom steht,
      - o: die ganze Zahl 2 oder 3 bedeutet und
      - R⁴ und R⁵: gleich oder verschieden sind und unabhängig voneinander für
      1. Wasserstoffatom,
      2. Phenyl oder
      3. Morpholin stehen,
A für eine kovalente Bindung oder -O- steht,
B für eine kovalente Bindung steht und
X für -CH=CH- steht.

Insbesondere bevorzugt sind die Verbindungen 2-(Biphenyl-4-sulfonylamino)-4-(naphthalen-1-yl-carbamoyl)-buttersäure, 2-(Biphenyl-4-sulfonylamino )-4-( naphthalen-2-yl-carbamoyl)-buttersäure, 2-( Biphenyl-4-sulfonylamino)-4-(2-phenylamino-ethylcarbamoyl)-buttersäure, 2-(4'-Chlor-biphenyl-4-sulfonylamino)-4-(3-morpholin-4-yl-propylcarbamoyl)-buttersäure, 4-(3-(4-(Biphenyl-4-sulfonylamino)-4-carboxy-butyrylamino)-propyl)-morpholin-4-ium-trifluor-acetat, 2-(4'-Chlorbiphenyl-4-sulfonylamino)-5-(2,3-dihydroindol-1-yl)-5-oxo-pentansäure, 2-(4'-Brombiphenyl-4-sulfonylamino)-5-(2,3-dihydroindol-1-yl)-5-oxo-pentansäure, 2-(4'-Chlorbiphenyl-4-sulfonylamino)-5-(5-fluor-2,3-dihydroindol-1-yl)-5-oxo-pentansäure, 2-(4'-Brom-biphenyl-4-sulfonylamino)-5-(5-fluor-2,3-dihydroindol-1-yl)-5-oxo-pentansäure, 5-(5-Fluor-2,3-dihydroindol-1-yl)-5-oxo-2-(4'-Pyrrolidin-1-yl-biphenyl-4-sulfonylamino)-pentansäure oder 5-(5-Fluor-2,3-dihydroindol-1-yl)-2-(4'-morpholin-4-yl-biphenyl-4-sulfonylamino)-5-oxo-pentansäure.

Mit dem Begriff "R⁴ und R⁵ zusammen mit der ringständigen Aminogruppe einen 4-bis 7-gliedrigen Ring bilden und/oder eines der Kohlenstoffatome durch -O-, -S- oder - NH- ersetzt ist" werden Reste verstanden, die sich beispielsweise von Isoxazolidin, Morpholin, Isothiazolidin, Thiomorpholin, Pyrazolidin, Imidazolidin, Piperazin, Azetidin, Pyrrol, Pyrrolin, Pyrrolidin, Pyridin, Azepin, Piperidin, Pyrazol oder Diazepin ableiten. Unter dem Begriff "Halogen" wird Fluor, Chlor, Brom oder Jod verstanden. Unter dem Begriff "Alkyl" werden Kohlenwasserstoffreste verstanden deren Kohlenstoffkette gerade oder verzweigt sind.
Die Ausgangsstoffe der chemischen Umsetzungen sind bekannt oder lassen sich nach literaturbekannten Methoden leicht herstellen.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, daß man (wie in Methode A, siehe Beispiele, beschrieben)
a) eine Aminocarbonsäure der Formel II, 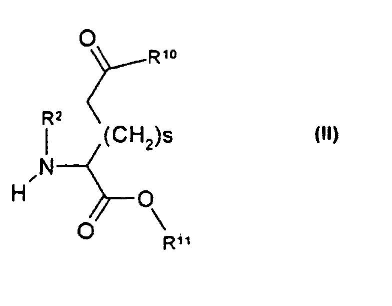 worin R¹¹ ein Wasserstoffatom oder eine in der Peptidchemie übliche Ester-Schutz-gruppe bedeutet, s die ganze Zahl Null, 1, 2 oder 3 bedeutet, R¹⁰ den Rest -OR¹² bedeutet, worin R¹² eine in Gegenwart von R¹¹ spaltbare Ester-Schutzgruppe oder ein Wasserstoffatom ist, oder R¹⁰ einen Rest -N(R⁶)-R⁷ bedeutet, worin R⁶ und R⁷ wie in Formel I definiert sind,
   mit einem Sulfonsäurederivat der Formel III, 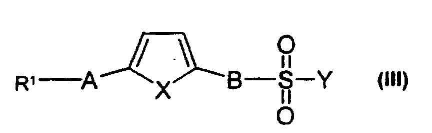 wobei R¹, X, A und B wie in Formel I definiert sind und Y ein Halogenatom, Imidazolyl oder OR¹³ bedeutet, worin R¹³ Wasserstoffatom, (C₁-C₆)-Alkyl, Phenyl oder Benzyl, welches gegebenenfalls substituiert ist, darstellt,
   in Gegenwart einer Base oder gegebenenfalls eines wasserentziehenden Mittels zu einer Verbindung der Formel IV, 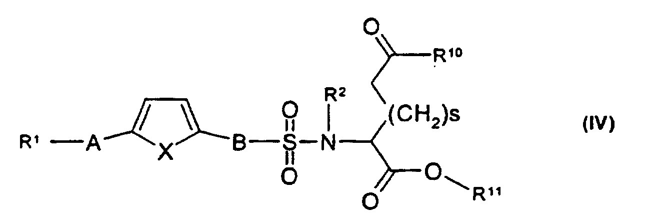 worin R¹, A, X, B, R², s, R¹⁰ und R¹¹ die oben genannte Bedeutung haben, umsetzt und anschließend eine eventuell im Rest R¹⁰ mitgeführte Schutzgruppe R¹² in Gegenwart der Schutzgruppe R¹¹ abspaltet, und danach den Rest -N(R⁶)-R⁷ nach entsprechender, aus der Peptidchemie bekannter Aktivierung der Carboxylgruppe einführt, und die mitgeführte Schutzgruppe R¹¹ abspaltet und die Verbindung der Formel I erhält, oder (wie in Methode B und C, siehe Beispiele, beschrieben)
b) ein Aminosäureanhydrid der Formel V, 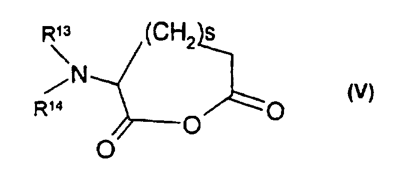 in der R¹³ ein Wasserstoffatom und R¹⁴ eine aus der Peptidchemie bekannte N-Schutzgruppe, wie z.B. Carbobenzyloxy (Z) darstellt, oder R¹³ und R¹⁴ eine cyclische N-Schutzgruppe, z.B Phthalimido bedeuten und s wie oben definiert ist, mit einem primären oder sekundären Rest -N(R⁶)-R⁷ im Sinne einer Ringöffnung zu einem Zwischenprodukt der Formel VI, 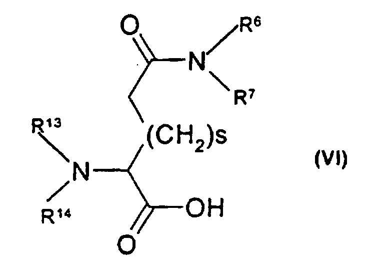 worin R¹³, R¹⁴, s, R⁶ und R⁷ die obengenannte Bedeutung haben, umsetzt, wobei diese Ringöffnung in Abhängigkeit von Schutzgruppe und Reaktionsbe-dingungen (vergl. X. Huang, X. Luo, Y. Roupioz, J. W. Keillor, J. Org. Chem. 1997, 62, 8821-8825) in der Regel regioselektiv das in Formel VI gezeigte Isomere liefert, das beim Auftreten von Regioisomerengemischen durch Kristallisation oder Chromatographie weiter angereichert werden kann, anschließend die mitgeführte Schutzgruppe R¹³ und/oder R¹⁴ unter Feisetzung des Amines spaltet, und danach wie unter a) beschrieben, mit einem Sulfonsäurederivat der Formel III eine N-Sulfonierung durchführt, die zu einem Produkt der Formel I führt, oder
c) eine nach den Verfahren a) oder b) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
d) die nach den Verfahren a), b) oder c) hergestellte Verbindung der Formel I entweder in freier Form isoliert oder im Falle des Vorliegens von sauren oder basischen Gruppen in physiologisch verträgliche Salze umwandelt.

Als geeignete Schutzgruppen werden dafür vorzugsweise die in der Peptidchemie gebräuchlichen N-Schutzgruppen verwendet, beispielsweise Schutzgruppen vom Urethan-Typ, Benzyloxycarbonyl (Z), t-Butyloxycarbonyl (Boc), 9-Fluorenyloxycarbonyl (Fmoc), Allyloxycarbonyl (Alloc) oder vom Säureamid-Typ insbesondere Formyl, Acetyl oder Trifluoracetyl sowie vom Alkyl-Typ beispielsweise Benzyl.

Als Ausgangsprodukte zur Darstellung der Sulfonsäurederivate der Formel III dienen bevorzugt Sulfonsäuren oder deren Salze der Formel VII, beispielsweise 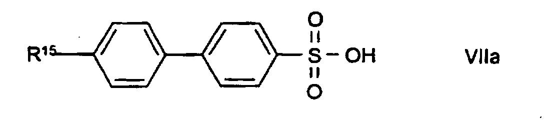 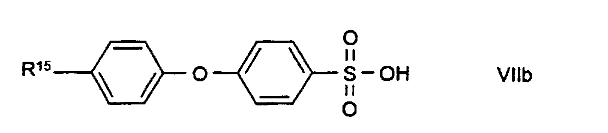 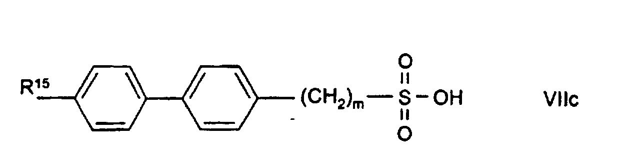 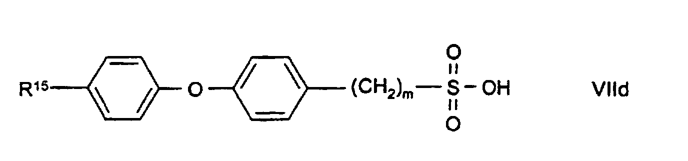 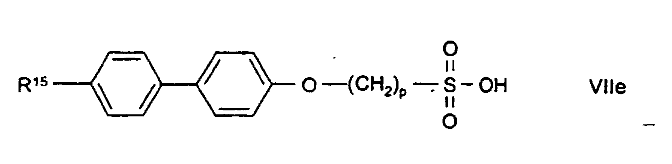 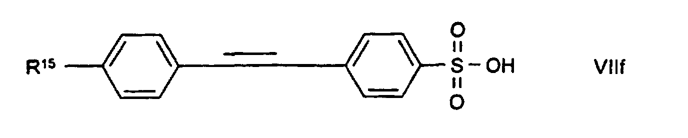 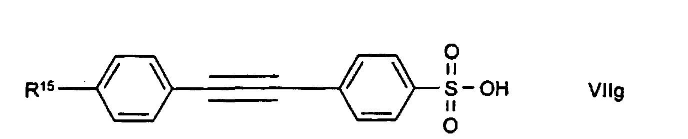 wobei R¹⁵ ein für R¹ unter 2.1. bis 2.14. beschriebener Rest bedeutet.
Sulfonsäurederivate der Formel III, die als R¹⁵ eine sekundäres oder cyclisches Amin vom Typ -N(R⁴)-R⁵ enthalten, stellt man vorzugsweise und in hohen Ausbeuten durch Pd-katalysierte Substitution eines Bisaryl-halogenides, bevorzugt eines Bromides durch ein sekundäres Amin in Anlehnung an bekannte Literaturvorschriften (vergl. A.S. Guram, R.A. Rennels, S.L. Buchwald; Angew. Chem. 1995, 107, 1456-1459) und anschließende Sulfochlorierung mittels Chlorsulfonsäure dar. Dabei wird die Sulfochloridfunktion durch den dirigierenden Effekt der Amingruppe bevorzugt in die gewünschte para-Position gelenkt.
Den vorteilhaft verwendeten Katalysator Dichlorobis(tritolylphosphin)-palladium(II) kann man analog zu R.F. Heck in "Palladium Reagents in Organic Syntheses", Academic Press, London (1985), S. 18 ausgehend von Tri-o-tolylphosphin, Palladium-(II)-chlorid und LiCl in Methanol herstellen.

Zur Herstellung der Arylsulfonsäuren der Formeln VIIa und Vllb bedient man sich vorzugsweise der im Houben-Weyl "Methoden der Organischen Chemie" Band 9, S. 540-546 beschriebenen Sulfonierungsverfahren mit konzentrierter Schwefelsäure ggf. in Gegenwart eines Katalysators, Schwefeltrioxids und seinen Additionsverbindungen oder Halogensulfonsäuren, wie Chlorsulfonsäure. Besonders im Falle der Diphenylether der Formel VIIb hat sich die Verwendung von konzentrierte Schwefelsäure und Essigsäureanhydrid als Lösemittel (vergl. C.M. Suter, J. Am. Chem. Soc. 53 (1931) 1114), oder die. Umsetzung mit überschüssiger Chlorsulfonsäure (J.P. Bassin, R. Cremlyn und F. Swinbourne; Phosphorus, Sulfur and Silicon 72 (1992) 157) bewährt. Sulfonsäuren gemäß der Formeln VIIc, VIId, oder Vlle lassen sich in an sich bekannter Weise herstellen, in dem man das entsprechende Arylalkylhalogenid mit Sulfiten wie Natriumsulfit oder Ammoniumsulfit in wäßriger oder wäßrig/alkoholischer Lösung umsetzt, wobei die Umsetzung in Gegenwart von Tetraorganoammoniumsalzen wie Tetrabutylammoniumchlorid beschleunigt werden kann.

Als Sulfonsäurederivate gemäß Formel III finden insbesondere die Sulfonsäurechloride Verwendung. Zu ihrer Herstellung werden die entsprechenden Sulfonsäuren, auch in Form ihrer Salze wie Natrium-, Ammonium- oder Pyridiniumsalze in bekannter Weise mit Phosphorpentachlorid oder Thionylchlorid ohne oder in Gegenwart eines Lösemittels wie Phosphoroxytrichlorid oder eines inerten Lösemittels wie Methylenchlorid, Cyclohexan oder Chloroform im allgemeinen bei Reaktionstemperaturen von 20°C bis zum Siedepunkt des verwendeten Reaktionsmediums umgesetzt. Vorteilhaft läßt sich auch die direkte Sulfochlorierung der entsprechenden Aromaten mit Chlorsulfonsäure, Sulfurylchlorid oder Pyrosulfurylchlorid durchführen (Houben-Weyl, Methoden der Organischen Chemie, Band 9 (1995), Seiten 572 - 579)

Die Umsetzung der Sulfonsäurederivate der Formel III mit den Aminosäuren der Formel II gemäß Verfahrensvarianten a) oder b) verläuft vorteilhaft nach Art der Schotten-Baumann-Reaktion. Als Basen eignen sich dafür besonders Alkalihydroxide wie Natriumhydroxid, aber auch Alkaliacetate, -hydrogencarbonate, -carbonate und Amine. Die Umsetzung findet in Wasser oder in einem mit Wasser mischbaren oder nichtmischbaren Lösemittel wie Tetrahydrofuran (THF), Aceton, Dioxan oder Acetonitril statt, wobei die Reaktionstemperatur im allgemeinen von -10°C bis 50°C gehalten wird. Für den Fall, daß die Reaktion im wasserfreien Medium durchgeführt wird, findet vor allem Tetrahydrofuran oder Methylenchlorid, Acetonitril oder Dioxan in Gegenwart einer Base, wie Triethylamin, N-Methylmorpholin, N-Ethyl- oder Diisopropylethylamin Verwendung, eventuell in Gegenwart von N,N-Dimethylaminopyridin als Katalysator.

In einer anderen Variante, besonders bei Verwendung polarer Ausgangsprodukte, die in ungeschützter Form vorliegen, kann man die Aminocarbonsäuren der Formel II zuerst mit Hilfe eines Silylierungsmittels wie Bis-trimethylsilyltrifluoracetamid (BSTFA) in ihre silylierte Form überführen und sie dann mit Sulfonsäurederivaten zu Verbindungen der Formel IV umsetzen.

Die Herstellung physiologisch verträglicher Salze aus zur Salzbildung befähigten Verbindungen der Formel I, einschließfich deren stereoisomeren Formen, erfolgt in an sich bekannter Weise. Die Carbonsäuren bilden mit basischen Reagenzien wie Hydroxiden, Carbonaten, Hydrogencarbonaten, Alkoholaten sowie Ammoniak oder organischen Basen, beispielsweise 2-Amino-2-(hydroxymethyl)-1,3-propandiol (Tromethamin), Trimethyl- oder Triethylamin, Ethanolamin oder Triethanolamin oder auch basischen Aminosäuren, etwa Lysin, Ornithin oder Arginin, stabile Alkali-, Erdalkali- oder gegebenenfalls substituierte Ammoniumsalze. Salze der Verbindung der Formel I, die mit den genannten organischen Basen gebildet werden, zeigen eine hohe Wasserlöslichkeit. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Phosphor-, Methansulfon-, Benzolsulfon-, p-Tofuolsulfon-, 4-Brombenzol-sulfon-, Essig-, Oxal-, Wein-, Trifluormethylsulfon-, Cyclohexylamidosulfon-, Bernstein- oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe und Therapie all solcher Erkrankungen, an deren Verlauf Matrix-abbauende Metalloproteinasen beteiligt sind. Dazu gehören degenerative Gelenkerkrankungen wie Osteoarthrosen, rheumatoide Arthritis, Spondylosen, Knorpelschwund nach Gelenktrauma oder längerer Gelenksruhigstellung, z.B. nach Meniskus- oder Patellaverietzungen oder Bänderrissen. Ferner gehören dazu auch Erkrankungen des Bindegewebes wie Kollagenosen, Periodontalerkrankungen, die sogar zum Verlust der Zähne führen können, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels (wie Osteoporose). Der medizinische Einsatz der erfindungsgemäßen Verbindungen der Formel I kann bei Gefäßkrankheiten, z.B. Blutgefäßverschlüssen, atherosklerotischen Plaques oder Aneurysmen indiziert sein, besonders bei drohender Ruptur, bzw. bei Stenosen jedweder Pathogenese. Weiterhin eignen sich die Verbindungen der Formel I zur Behandlung von Entzündungen, einschließlich Wunden und Geschwüren, insbesondere auch solcher der Haut, Krebserkrankungen, insbesondere auch zur Blockierung bzw. Eindämmung der Metastasenbildung und -ausbreitung, und auch bei Mammakarzinom. Die Behandlung von Kachexie, Anorexie, septischem Schock, Periodontose und Periodontitis sind weitere medizinische Anwendungsgebiete für die erfindungsgemäßen Verbindungen.

Die erfindungsgemäßen Arzneimittel werden im allgemeinen oral oder parenteral verabreicht. Die rektale oder transdermale Applikation ist auch möglich.

Die Erfindung betrifft auch ein Verfahren zur Herstellung eines Arzneimittels, das dadurch gekennzeichnet, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien Magnesium-carbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.
Für die Behandlung eines erwachsenen, etwa 70 kg schweren Patienten sind - je nach Wirksamkeit der Verbindungen gemäß Formel I - Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in Intervallen erfolgen.

Die der Erfindung zugrunde liegenden Verbindungen wurden durch Kemresonanz-, und Massenspektroskopie identifiziert, wobei im Falle des Vorliegens regio- oder diastereoisomerer Formen neben ¹H- auch ¹³C- sowie multidimensionale NMR-Methoden zum eindeutigen Strukturbeweis eingesetzt wurden. ¹H-NMR-Spektren sind an einem 400-MHz-Gerät der Firma Bruker aufgenommen worden, in der Regel mit Tetramethylsilan (TMS) als internem Standard und bei Raumtemperatur (RT). Endprodukte werden in der Regel durch massenspektroskopische Methoden (FAB-, ESI-MS mit positiver oder negativer Ionisierung) bestimmt. Temperaturangaben sind in Grad Celsius, RT bedeutet Raumtemperatur (22 °C bis 26 °C). Verwendete Abkürzungen sind entweder erläutert oder entsprechen den üblichen Konventionen.

### Herstellungsbeispiele

### Methode A)

### Beispiel 5 4-(3-(4-(Biphenyl-4-sulfonylamino)-4-carboxy-butyrylamino)-propyl)-morpholin-4-ium-Trifluoracetat

### Stufe 1: Sulfonierung von Glutamyl-tert.-Butylester

3 g (0,0148 mol) L-Glu-OtBu wurden in 29,5 ml 0,5 N NaOH und 250 ml Tetrahydrofuran (THF) gelöst, im Eisbad auf 0 °C gekühlt, und gleichzeitig (unter pH-Konstanthaltung bei pH 8,5) wurde eine Lösung von 4,476 g (0,0177 mol) Biphenylsulfonylchlorid in 35 ml THF und 29,52 ml einer wässrigen 0,5 N NaOH in etwa 30 Minuten (min) lang zugetropft. Nach Entfernen des Eisbades wurde 3 Stunden (h) bei Raumtemperatur nachgerührt und die Beendigung der Reaktion wurde durch Dünnschichtchromatographie (DC) verfolgt. Man entfernte THF unter reduziertem Druck, extrahierte einmal mit Diethylether, säuerte mit 1 N HCl bis pH 1,5 an, extrahierte das Produkt mehrfach mit Ethylacetat, trocknete und entfernte das Lösemittel unter reduziertem Druck. Es verblieben 2,89 g an Produkt, das ohne weitere Reinigung weiter umgesetzt werden konnte.

### Stufe 2: Überführung in das Amid

0,25 g (0,596 mmol) des Produktes von Stufe 1 wurden in 50 ml absoluten THF gelöst, 0,119 g (0,63 mmol) EDC (1-(3-Dimethylaminopropyl)-3-ethyl-carbodiimid-Hydrochlorid) und 0,0845 g (0,55 mmol) 1-Hydroxybenzotriazol-Hydrat wurden zugesetzt, 30 min gerührt, dann wurde vorsichtig 0,268 ml (1,79 mmol) N-(3-Aminopropyl)-morpholin zugegeben, und etwa 8 h nachgerührt. Man nahm mit Wasser auf, säuerte mit 1 N HCl bis pH 2 an, extrahierte mehrmals mit Ethylacetat, wusch mit verdünnter NaCl , trocknete und engte ein. Ausbeute : 250 mg. Ein weitere Reinigung konnte durch Chromatographie an Kieselgel mittels Dichlormethan/ Methanol 9:1 erfolgen.

### Stufe 3: Abspaltung der Schutzgruppe

0,11 g des Produktes von Stufe 2 wurden in 5 ml Trifluoressigsäure und 5 ml Dichlormethan gelöst und unter Schutzgas bis zur Beendigung der Reaktion (DC-Kontrolle) etwa 3 h bei Raumtemperatur gerührt. Nach Einengen wurde mehrfach mit Dichlormethan abgeschleppt und unter reduziertem Druck wurde getrocknet. Es verblieben 0,11 g der Verbindung des Beispiels 5 als Trifluoracetat.

### Methode B)

### Beispiel 6 2-(Biphenyl-4-sulfonylamino)-5-(2,3-dihydro-indol-1-yl)-5-oxopentancarbonsäure

### Stufe 1: Umsetzung von Phthaloyl-L-glutamylanhydrid mit Dihydroindol

1,5 g (0,0058 mol) Phthaloyl-L-glutamylanhydrid wurden in 15 ml absoluten Dioxan gelöst, und eine Lösung von 0,813 ml (0,0073 mol) Dihydroindol in 15 ml Dioxan wurde innerhalb von 10 min zugetropft. Man erhitzte bis zur Beendigung der Reaktion (etwa 5 h, DC-Kontrolle) auf 40 °C, engte ein, und schleppte mehrmals unter vermindertem Druck mit Dichlormethan ab. Ausbeute: 2,7 g (Produkt erithielt etwa 15 mol % Dioxan)

### Stufe 2: Abspaltung der Schutzgruppe

2,43 g des Produktes von Stufe 1 wurden in 30 ml Ethanol gelöst und es wurde 0,39 ml (0,0080 mol) Hydrazin-Hydrat zugegeben. Man rührte 3 h bei 80 °C, engte unter reduziertem Druck bis zur Trockene ein, nahm den Rückstand mit 50 ml 25%-iger wäßriger Essigsäure auf und erhitzte für etwa 10 min auf 80 °C. Dann kühlte man im Eisbad ab und saugte vom entstandenen Niederschlag ab, nahm den Niederschlag erneut mit Essigsäure auf und wiederholte diese Vorgehensweise. Der verbleibende Phthalylhydrazid-Rückstand wurde verworfen. Die vereinigten Filtrate wurden eingeengt, und ergaben 0,7 g an Produkt.

### Stufe 3: Einführung des N-Arylsulfonylrestes

0,5 g (0,002 mol) des Produktes aus Stufe 2 wurden zusammen mit 0,45 g (0,0033 mol) Kaliumcarbonat in 50 ml THF/Wasser (1:1) gelöst und innerhalb von 20 min wurde eine Lösung von 0,61 g (0,0024 mol) Biphenylsulfonylchlorid, gelöst in 50 ml THF, zugetropft. Man rührte bei RT bis zur Beendigung der Reaktion (etwa 6 bis 8 h, DC-Kontrolle), extrahierte einmal mit Diethylether, säuerte mit 1 N HCl bis pH 1,5 an, extrahierte das Produkt mehrmals mit Ethylacetat, trocknete, und engte unter reduziertem Druck ein. Nach dem Trocknen verblieben 0,42 g des Produktes des Beispiels 6.

### Methode C)

### Beispiel 8 2-(4'-Chlor-biphenyl-4-sulfonylamino)-5-(2,3-dihydro-indol-1-yl)-5-oxopentansäure

### Stufe 1: Umsetzung von Cbz-Glu-Anhydrid mit 2,3-Dihydro-1H-indol

5 g (0,019 mol) L-Cbz-Glutamylanhydrid wurden zusammen mit 2,56 ml (dies entspricht 0,023 mol) 2,3-Dihydro-1H-indol in 100 ml absoluten Dimethylsulfoxid gelöst und 30 min bei Raumtemperatur (RT) sowie noch 30 min bei 40 °C bis zur Beendigung der Reaktion gerührt (DC-Kontrolle). Der Ansatz wurde in Wasser gegeben, mehrfach mit Ethylacetat extrahiert, die organische Phase wurde mit 1N HCl und mit gesättigter NaCl -Lösung gewaschen, getrocknet und eingeengt. Ausbeute: 7,07 g

### Stufe 2: Abspaltung der Cbz-Schutzgruppe

6,8 g (0,018 mol) des Produktes aus Stufe 1 wurden in 60 ml einer 33%-igen HBr/Eisessig-Lösung innerhalb von 30 min gelöst und bis zur Beendigung der Reaktion bei RT gerührt (etwa 15 h, DC-Kontrolle). Das nach Einengen unter verminderten Druck erhaltene Rohprodukt wurde mehrfach mit Dichlormethan abgeschleppt und mit Methanol/Wasser (10:1) aufgenommen, das Produkt wurde abfiltriert, getrocknet und nochmals in THF ausgerührt. Nach Absaugen und Trocknen unter verminderten Druck verblieben 2,13 g des kristallinen Hydrobromides mit einer Reinheit von 98% (nach HPLC).

Die reduktive Abspaltung der Schutzgruppe analog zu Beispiel 12, Stufe 4, ergab das Hydrochlorid von 2-Amino-5-(2,3-dihydro-indol-1-yl)-5-oxo-pentansäure in 85% Ausbeute.

### Stufe 3: 2-(4'-Chlor-biphenyl-4-sulfonylamino)-5-(2,3-dihydro-indol-1-yl)-5-oxopentansäure

0,33 g (1 mmol) des Produktes von Stufe 2 wurden in 60 ml THF/Wasser (1:1) gelöst und unter Verwendung eines Titroprozessors und 0,5 N NaOH wurde pH 12 eingestellt. Unter konstanten pH wurde innerhalb von etwa 45 min eine Lösung von 0,345 g (1,2 mmol) 4'-Chlor-biphenyl-4-sulfonytchlorid, gelöst in 30 ml THF, zugetropft. Man rührte noch 2 h bei pH 12 nach (DC-Kontrolle), versetzte mit 0,1 N HCl bis pH 2, extrahierte mehrfach mit Ethylacetat, wusch mit Wasser, trocknete und engte ein.
Ausbeute: 0,395 g vom Schmelzpunkt 182 °C und einer Reinheit von mehr als 92%.

Die als geringfügige Verunreinigung erkennbare 4-Chlorbiphenylsulfonsäure konnte durch Chromatografie oder Umfällen entfernt werden.

### Beispiel 12 5-(5-Fluoro-2,3-dihydro-indol-1-yl)-5-oxo-2-(4'-pyrrolidin-1-yl-biphenyl-4-sulfonylamino)-pentansäure

### Stufe 1: Biphenyl-4-yl-pyrrolidin

10,0 g (0,043 mol) 4-Brombiphenyl wurden zusammen mit 3,7 g (0,052 mol) Pyrrolidin und 6,2 g (0,0642 mol) Natrium-tertiär-butanolat in 600 ml Toluol suspendiert und 900 mg des Katalysators Dichlorobis(tritolylphosphin)-palladium(II) wurden zugegeben. Man erhitzte für 8 h am Rückfluß, kühlte ab, versetzte mit Wasser/Ethylacetat, wusch die organische Phase mit Wasser, trocknete über Natriumsulfat, und engte ein. Der Rückstand wurde in etwa 300 ml tertiär-Butylmethylether gelöst und durch Zugabe von 50 ml 1N etherischer HCl wurde das Hydrochlorid des Produktes gefällt.
Ausbeute: 6,0 g vom Schmelzpunkt höher als 125 °C (Zersetzung)

### Stufe 2: 4'-Pyrrolidin-1-yl-biphenyl-4-sulfonyl chlorid

6 g (0,023 mol) des Produkts von Stufe 1 wurde unter Kühlen und Schutzgas portionsweise in 11 ml Chlorsulfonsäure eingetragen und für 3,5 h auf 60 °C erhitzt. Man gab die Lösung auf Eis, brachte die Suspension durch Zugabe von festem Natriumbicarbonat auf pH 8, saugte das ausgefallene Produkt ab und trocknete unter vermindertem Druck.
Ausbeute: 7 g vom Schmelzpunkt höher als 282 °C (Zersetzung).

### Stufe 3: Umsetzung von Z-Glu-Anhydrid mit 5-Fluoro-2,3-dihydro-1H-indol

8,8 g (0,0334 mol) L-Z-Glutamylanhydrid wurden zusammen mit 5,5 g (0,04 mol) 5-Fluoro-2,3-dihydro-1H-indol in 150 ml absoluten Dimethylsulfoxid gelöst und bei RT bis zur Beendigung der Reaktion gerührt (etwa 3 h, DC-Kontrolle). Der Ansatz wurde in Wasser gegeben, mehrfach mit Ethylacetat extrahiert und die organische Phase wurde mit 1N HCl und mit gesättigten NaCl gewaschen, getrocknet und eingeengt.
Ausbeute: 13,2 g

### Stufe 4: Abspaltung der Z-Schutzgruppe

13 g (0,0325 mol) des Produktes aus Stufe 3 wurden in 450 ml Methanol und 33 ml 1N HCl gelöst, eine Spatelspitze Palladium auf Kohle zugesetzt, und bei 40 bar und 60 °C bis zur Beendigung der Reaktion hydriert (DC-Kontrolle). Das nach Filtration und Einengen erhaltene Rohprodukt wurde unter Rückfluß in THF aufgenommen, das nach Abkühlen verbleibende feste Hydrochlorid wurde abfiltriert, und unter vermindertem Druck getrocknet. Ausbeute: 7,1 g vom Schmelzpunkt höher als 212 °C

### Stufe 5: 5-(5-Fluoro-2,3-dihydro-indol-1-yl)-5-oxo-2-(4'-pyrrolidin-1-yl-biphenyl-4-sulfonylamino)- pentansäure

0,303 g ( 1 mmol) des Produktes von Stufe 4 wurden in 60 ml THF/Wasser (1:1) ) gelöst und unter Verwendung eines Titroprozessors (Modell 686, Firma Metrohm) wurde mittels 0,5 N NaOH ein pH von 12,0 eingestellt. Unter pH-Konstanthaltung wurde während etwa 45 min eine Lösung von 0,39 g (1,2 mmol) des Produktes aus Stufe 2, gelöst in 40 ml THF, zugetropft. Man rührte noch 2 h bei pH 12 nach (DC-Kontrolle), versetzte mit 0,1 N HCl bis ein pH von 2 erreicht wurde, extrahierte mehrfach mit Ethylacetat, wusch mit Wasser, trocknete, und engte ein. Das Produkt wurde durch Chromatographie an Kieselgel (Dichlormethan/Methanol 95:5) gereinigt. Ausbeute: 0,393 g vom Schmelzpunkt 216 °C

### Methode D)

### Beispiel 15 Tromethamin-Salz von 2-(4'-Chlor-biphenyl-4-sulfonylamino)-5-(2,3-dihydro-indol-1-yl)-5-oxo-pentansäure

104,5 g (0,21 mol) der Verbindung gemäß Beispiel 8 wurden in 1000 ml Ethanol suspendiert und auf 50 °C erwärmt. Dann wurden 25,4 g Tromethamin (2-Amino-2-(hydroxymethyl)-1,3-propandiol,TRIS) gelöst in 100 ml Wasser dazugegeben. Das Gemisch wurde kurz bis zum Rückfluß erhitzt und heiß filtriert. Das klare Filtrat ließ man auf RT abkühlen, wobei das Salz ausfiel. Es wurde abgesaugt und zweimal mit je 150 ml Ethanol/Wasser im Verhältnis von 9 zu 1 gewaschen und im Exsikkator getrocknet. Ausbeute: 93,9g (72%) Tromethamin-Salz.

### Beispiel 16 Tris-(2-hydroxy-ethyl)-ammonium-2-(4'-chlor-biphenyl-4-sulfonylamino)-5-(2,3-dihydro-indol-1-yl)-5-oxo-pentanoat

0,05 g des Produktes aus Beispiel 8 wurden in 3,18 g Aceton gelöst, 0,01 g Triethanolamin zugesetzt, die Lösung wurde filtriert und in einem offenen Erlenmeyer-Gefäß in eine DC-Kammer gestellt, deren Boden etwa 1 cm mit tert.-Butylmethylether bedeckt war. Die Kristallbildung setzte nach etwa 10 h ein und war nach 4 bis 5 Tagen vollständig. Man dekantierte das überstehende Lösemittel ab und trocknete das erhaltene Produkt unter verminderten Druck. Ausbeute: 0,06 g vom Schmelzpunkt 120 °C.

Die Beispiele 1 bis 5 der Tabelle 1 wurden nach Methode A) hergestellt, Beispiele 6 und 7 nach Methode B) und die Beispiele 8 bis14) nach Methode C). Das für die Einführung der Arylsulfonyl-Seitenkette des Beispieles 13 benötigte Ausgangsprodukt (4'-Morpholin-4-yl-biphenyl-4-sulfonylchlorid) wurde analog zu Methode C), Stufe 1 hergestellt.

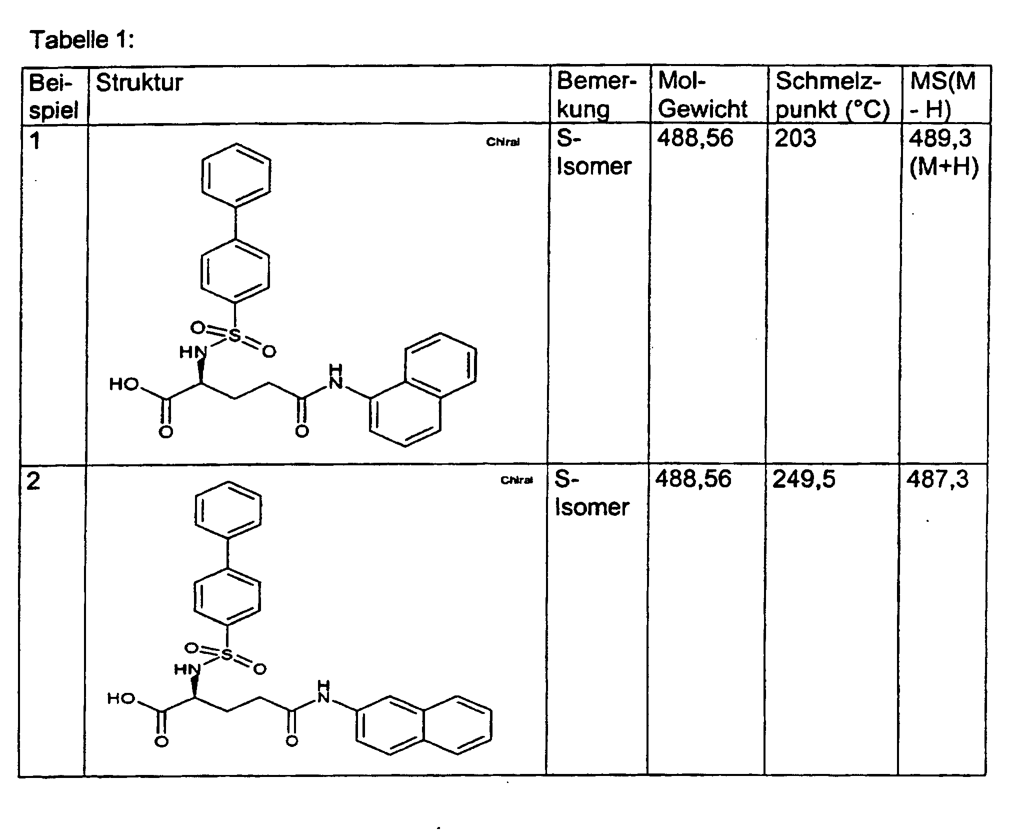

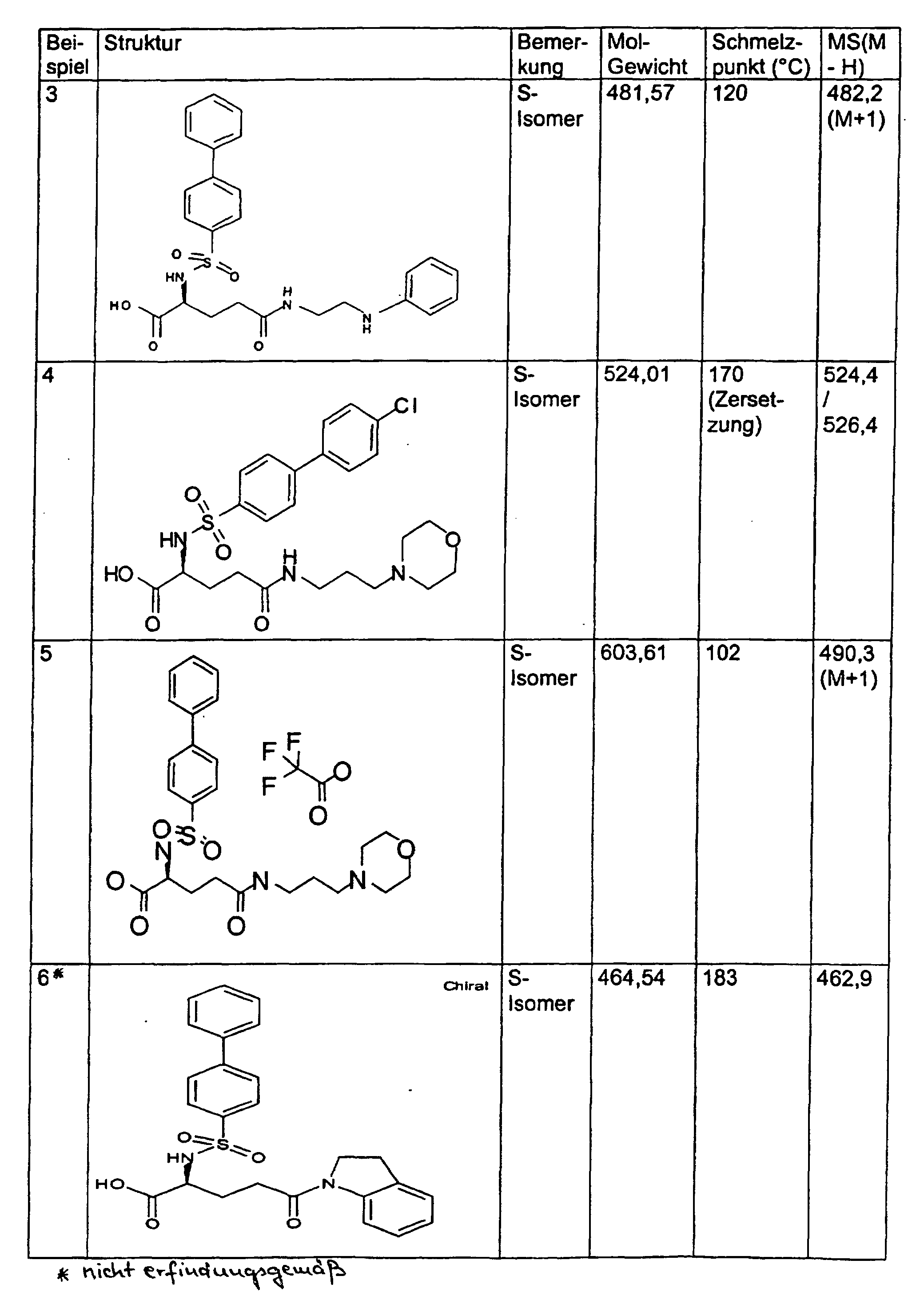

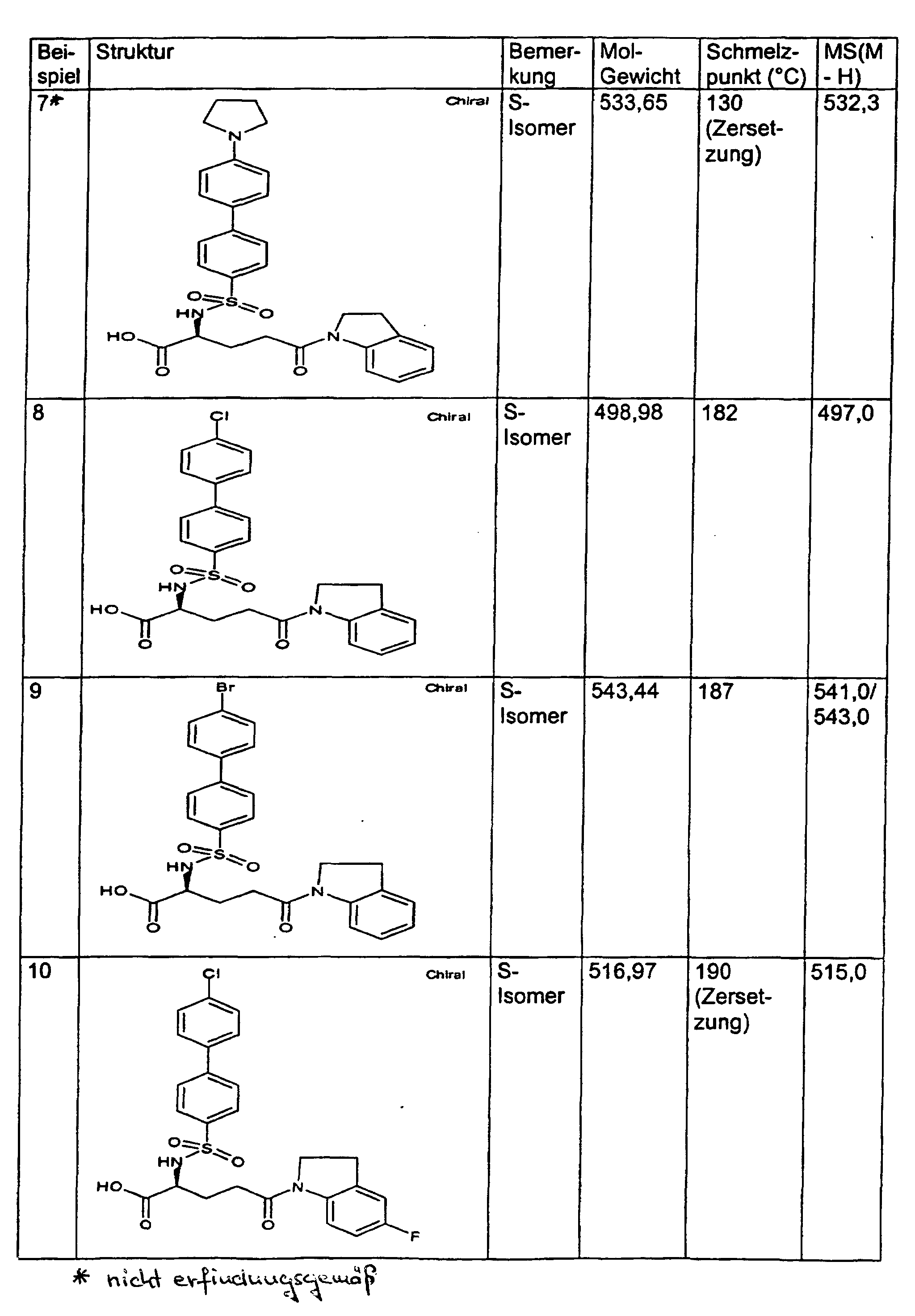

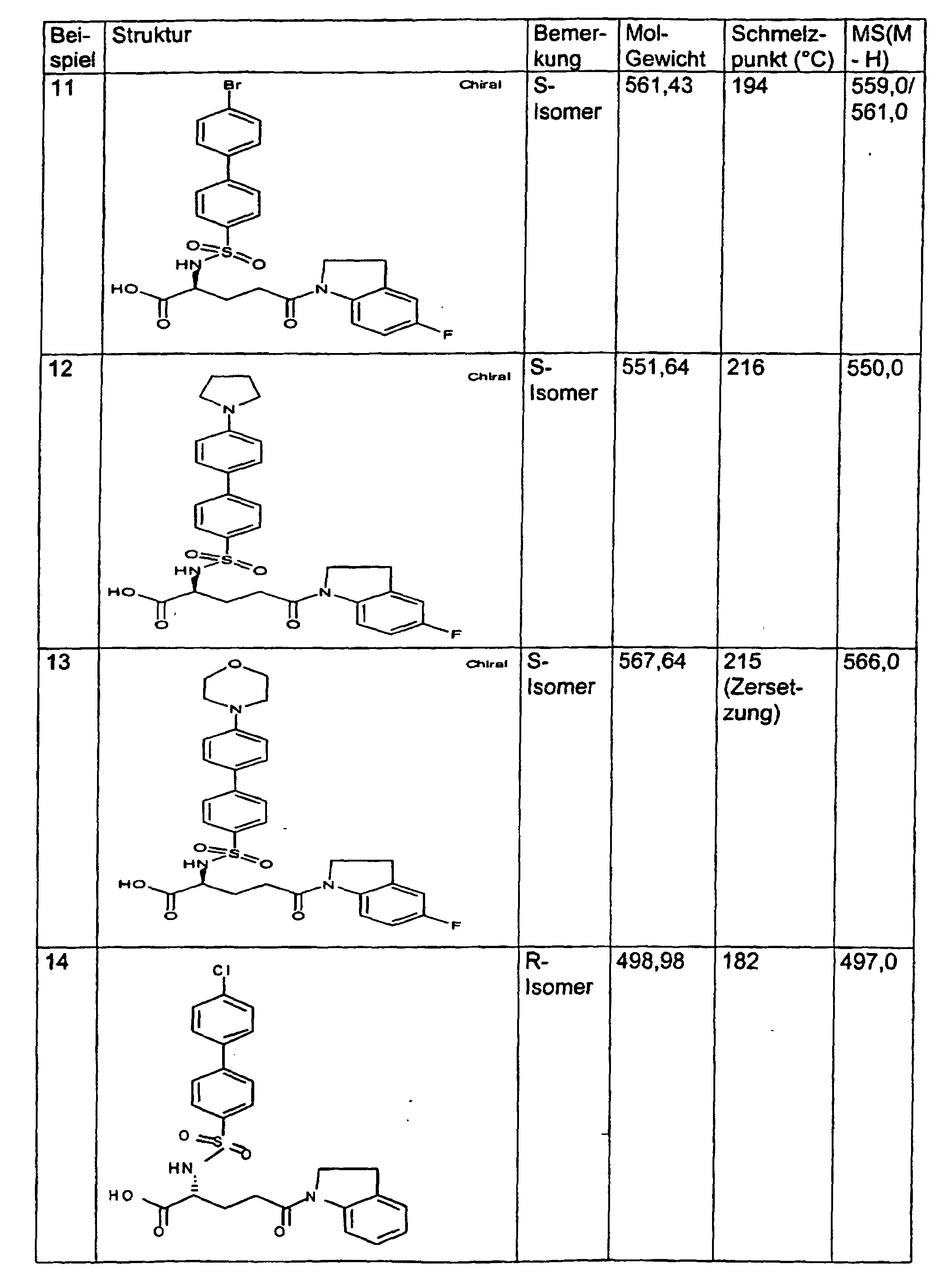

### Pharmakologische Beispiele

Darstellung und Bestimmung der enzymatischen Aktivität der katalytischen Domäne des humanen Stromelysins und der Neutrophilen-Kollagenase.

Die beiden Enzyme -Stromelysin (MMP-3) und Neutrophilen-Kollagenase (MMP-8) - wurden dargestellt nach Ye et al. (Biochemistry; 31 (1992) Seiten 11231-11235) oder Weithmann et al. (Inflamm Res 46(1997) 246-252 ). Zur Messung der Inhibitorwirkung auf die enzymatische Aktivität wurden 70 µl Pufferlösung, und 10 µl Enzymlösung mit 10 µl einer 10%igen (v/v) wäßrigen Dimethylsulfoxid-Lösung, die gegebenenfalls den Enzyminhibitor enthielt, für 15 Minuten inkubiert. Nach Zugabe von 10 µl einer 10%igen (v/v) wäßrigen Dimethylsulfoxid-Lösung, die 1 mmol/l des Substrates enthielt, wurde die Enzymreaktion fluoreszenzspektroskopisch verfolgt (328 nm (ex) / 393 nm(em)).

Die Enzymaktivität wurde dargestellt als Extinktionszunahme/Minute. Die in Tabelle 2 aufgeführten IC₅₀-Werte wurden als diejenige Inhibitorkonzentrationen ermittelt, die jeweils zu einer 50%igen Inhibierung des Enzyms führten. Die Pufferlösung enthielt 0,05% Brij (Sigma, Deisenhofen, Deutschland) sowie 0,1 mol/l Piperazin-N,N'-bis[2-ethan-sulfonsäure]/NaOH, 0,1 mol/l NaCl, 0,01 mol/l CaCl₂ und 0,1 mol/l Piperazin-N,N'-bis[2-ethan-sulfonsäure] (pH=6,5).

Die Enzymlösung enthielt 5 µg/ml einer der nach Ye et al. dargestellten Enzymdomänen. Die Substratlösung enthielt 1 mmol/l des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-NH₂ (Bachem, Heidelberg, Deutschland).

**Tabelle 2:**

| Beispiel | MMP-3 IC₅₀ [µmol/l] | MMP-8 IC₅₀ [µmol/l] |
|---|---|---|
| 1 | 0,1 | 0,01 |
| 2 | 0,2 | 0,02 |
| 3 | 0,2 | 0,02 |
| 4 | 0,1 | 0,02 |
| 5 | 0,1 | 0,006 |
| 6* | 0,5 | 0,02 |
| 7* | 0,04 | 0,004 |
| 8 | 0,1 | 0,01 |
| 9 | 0,1 | 0,02 |
| 10 | 0,1 | 0,02 |
| 11 | 0,1 | 0,02 |
| 12 | 0,03 | 0,002 |
| 13 | 0,1 | 0,01 |
| 14 | 0,05 | 0,01 |

| | | |
|---|---|---|
| * nicht erfindungsgemäß | | |

Bestimmung der Löslichkeit in Wasser der freien Säure von 2-(4'-Chlor-biphenyl-4-sulfonylamino)-5-(5-fluor-2,3-dihydroindol-1-yl)-5-oxo-pentansäure gemäß Beispiel 8 und dessen Tromethaminsalzes gemäß Beispiel 15
1. Eingesetzte Materialien
   1.1 Prüfsubstanzen
      a) Verbindung gemäß Beispiel 8; 2-(4'-Chlor-biphenyl-4-sulfonylamino)-5-(5-fluor-2,3-dihydroindol-1-yl)-5-oxo-pentansäure
      b) Verbindung gemäß Beispiel 15; Tromethaminsalz von 2-(4'-Chlor-biphenyl-4-sulfonylamino)-5-(5-fluor-2,3-dihydroindol-1-yl)-5-oxo-pentansäure
   1.2 Reagenzien
      - Wasser: entionisiert, - Acetonitril: LiChrosolv (Merck), - Diethylamin: zur Synthese (Merck), - Essigsäure: konzentriert und 1N (Merck)
2. Experimentelle Durchführung
   2.1 2-(4'-Chlor-biphenyl-4-sulfonylamino)-5-(5-fluor-2,3-dihydroindol-1-yl)-5-oxopentansäure
      In einem 100-ml-Erlenmeyerkolben wurden etwa 50 mg Prüfsubstanz gemäß 1.1 a) vorgelegt. Nach Zugabe von 10 ml Wasser wurde bei 25 °C gerührt. Nach 2 Stunden (h) sowie nach 3 h wurden Aliquots entnommen und zentrifugiert. Je 1 ml des Überstandes wurde mit mobiler Phase auf 10 ml verdünnt. Die Konzentration der aktiven Komponente wurde mittels HPLC gegen einen externen Standard bestimmt. Wiederholung des Experimentes.
   2.2 Tromethaminsalz von 2-(4'-Chlor-biphenyl-4-sulfonylamino)-5-(5-fluor-2,3-dihydroindol-1-yl)-5-oxo-pentansäure
      In einem 100-ml-Erlenmeyerkolben wurden etwa 800 mg des Tromethaminsalzes gemäß 1.1 b) vorgelegt. Nach Zugabe von 10 ml Wasser wurde bei 25 °C gerührt. Nach 2 h sowie nach 3 h wurden Aliquots entnommen und zentrifugiert. Je 1 ml des Überstandes wurde mit mobiler Phase auf 100 ml verdünnt. Die Konzentration der aktiven Komponente wurde mittels HPLC gegen einen externen Standard bestimmt. Wiederholung des Experimentes.
3. Analytik
   - Instrument:: HPLC-Anlage Gynkotek
   - Säule:: Stationäre Phase: ChiraDex 5µm, Merck Dimensionen: 250 mm x 4.0 mm
   - Mobile Phase:: Acetonitril 440 ml; Wasser 560 ml; Diethylamin 1 ml; Mit Essigsäure auf pH 6,4 eingestellt.
   - Injektionsvolumen:: 10 µl
   - Fluß:: 0,8 ml/min
   - Säulentemperatur:: 40°C
   - Detektion:: UV bei 261 nm
5. Ergebnisse

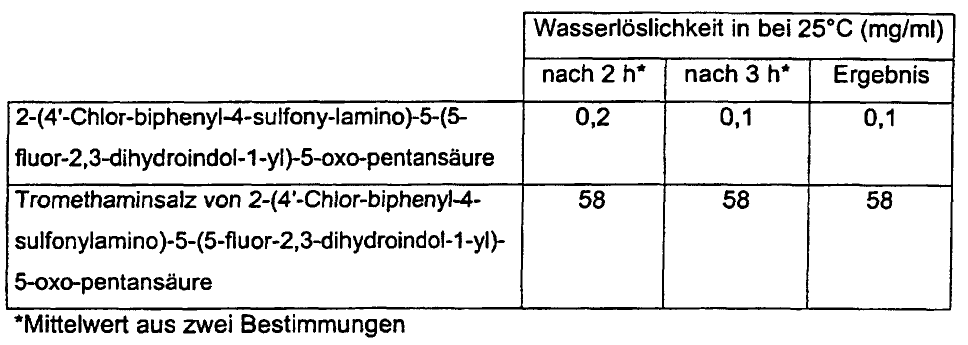

Der zeitabhängige Unterschied in den Löslichkeitswerten von 2-(4'-Chlor-biphenyl-4-sulfonylamino)-5-(5-fluor-2,3-dihydroindol-1-yl)-5-oxo-pentansäure könnte auf Übersättigungs-/Sättigungsphänomene zurückführbar sein. Als Ergebnis wird der nach 3 h erhaltene Datenwert herangezogen.

Die Wasserlöslichkeit des Tromethaminsalzes von 2-(4'-Chlor-biphenyl-4-sulfonylamino)-5-(5-fluor-2,3-dihydroindol-1-yl)-5-oxo-pentansäure ist etwa um den Faktor 600 höher als diejenige der freien Säure von 2-(4-Chlor-biphenyl-4-sulfonylamino)-5-(5-fluor-2,3-dihydroindol-1-yl)-5-oxo-pentansäure.

## Patentansprüche

1. Verbindung der Formel I 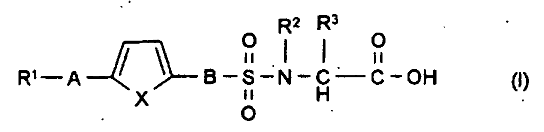 und/oder eine stereoisomere Form der Verbindung der Formel I und/oder ein physiologisch verträgliches Salz der Verbindung der Formel I, wobei
R¹ für
1. Phenyl,
2. Phenyl, welches ein- oder zweifach substituiert ist durch
2.1. (C₁-C₆)-Alkyl, gerade, cyclisch oder verzweigt,
2.2. Hydroxy,
2.3. (C₁-C₆)-Alkyl-C(O)-O-,
2.4. (C₁-C₆)-Alkyl-O-,
2.5. (C₁-C₆)-Alkyl-O-(C₁-C₄)-Alkyl-O-,
2.6. Halogen,
2.7. -CF₃,
2.8. -CN,
2.9. -NO₂,
2.10. HO-C(O)-,
2.11. (C₁-C₆)-Alkyl-O-C(O)-,
2.12. Methylendioxo,
2.13. R⁴-(R⁵)N-C(O)-,
2.14. R⁴-(R⁵)N-, oder
2.15. Rest eines Heterocycluses aus der nachfolgenden Gruppe: Isoxazolidin, Morpholin, Isothiazolidin, Thiomorpholin, Pyrazolidin, Imidazolidin, Piperazin, Azetidin, Pyrrol, Pyrrolin, Pyrrolidin, Pyridin, Azepin, Piperidin, Oxazol, Isoxazol, Imidazol, Pyrazol, Thiazol, tsothiazol, Diazepin, Thiomorpholin, Pyrimidin und Pyrazin, der unsubstituiert oder wie für R¹ unter 2.1 bis 2.14 beschrieben substituiert ist, oder
3. einen Heteroaromaten aus der nachfolgenden Gruppe 3.1. bis 3.15., der unsubstituiert oder wie für R¹ unter 2.1 bis 2.14 beschrieben substituiert ist,
3.1. Pyrrol,
3.2. Pyrazol,
3.3. Imidazol,
3.4. Triazol,
3.5. Thiophen,
3.6. Thiazol,
3.7. Oxazol,
3.8. Isoxazol,
3.9. Pyridin,
3.10. Pyrimidin,
3.11. Indol,
3.12. Benzothiophen,
3.13. Benzimidazol,
3.14. Benzoxazol oder
3.15. Benzothiazol steht,
R², R⁴ und R⁵ gleich oder verschieden sind und für
1. Wasserstoffatom,
2. (C₁-C₆)-Alkyl-,
3. HO-C(O)-(C₁-C₆)-Alkyl-,
4. Phenyl-(CH₂)ₙ-, worin Phenyl unsubstituiert oder ein- oder zweifach wie für R¹ unter 2.1. bis 2.14. beschrieben substituiert ist und n die ganze Zahl Null, 1 oder 2 darstellt, oder
5. Picolyl stehen oder
6. R⁴ und R⁵ zusammen mit der ringständigen Aminogruppe einen 4- bis 7-gliedrigen Ring bilden, worin gegebenenfalls eines der Kohlenstoffatome durch -O-, -S- oder -NH- ersetzt ist,
R³ für
1. -(C₁-C₄)-Alkyl-C(O)-N(R⁶)-R⁷, worin R⁶ und R⁷ zusammen mit dem Stickstoff, an den sie gebunden sind einen Rest der Teilformeln IIa, IIb oder IIe bilden, 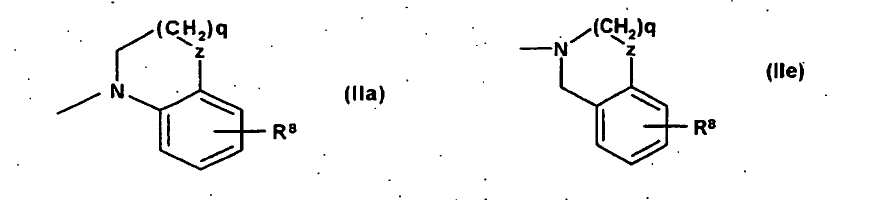 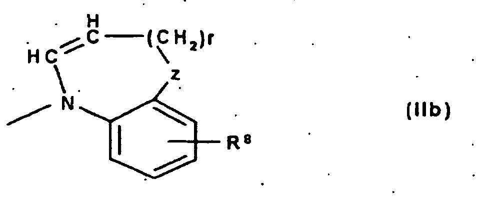 wobei in den Teilformeln IIa, IIb und IIe
q die ganze Zahl Null, 1 oder 2 bedeutet und
r die ganze Zahl Null oder 1 bedeutet,
Z ein Kohlenstoff-, Stickstoff-, Sauerstoff- oder Schwefelatom oder eine kovalente Bindung bedeutet, und
R⁸ Wasserstoffatom bedeutet oder die für R¹ oben unter 2.1 bis 2.14 beschriebene Bedeutung hat,
2. -(C₁-C₄)-Alkyl-C(O)-Y,
worin Y einen Rest der Teilformeln IIc oder IId bedeutet, 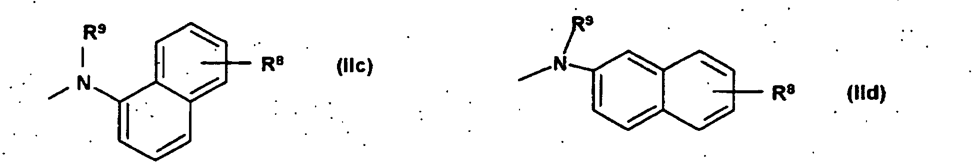 wobei in den Teilformeln IIc und IId
R⁸ Wasserstoffatom bedeutet oder die für R¹ oben unter 2.1 bis 2.14 beschriebene Bedeutung hat und
R⁹ für
2.1 Wasserstoffatom
2.2 (C₁-C₆)-Alkyl-,
2.3 HO-C(O)-(C₁-C₆)-Alkyl-,
2.4 Phenyl-(CH₂)ₙ-, worin Phenyl unsubstituiert oder ein- oder zweifach wie für R¹ unter 2.1 bis 2.14 beschrieben substituiert ist und n die ganze Zahl Null, 1 oder 2 darstellt, oder
2.5 Picolyl steht, oder
3. -(C₁-C₄)-Alkyl-C(O)-N(R⁹)-(CH₂)₀-N(R⁴)-R⁵, wobei
R⁹ die oben genannte Bedeutung hat,
o die ganze Zahl 2, 3, 4 oder 5 bedeutet und
R⁴ und R⁵ zusammen mit der ringständigen Aminogruppe einen 4-bis 7-gliedrigen Ring bilden aus der Gruppe Isoxazolidin, Morpholin, Isothiazolidin, Thiomorpholin, Pyrazolidin, Imidazolidin, Piperazin, Azetidin, Pyrrolin, Pyrrolidin, Azepin, Piperidin oder Diazepin, steht,
A für
a) eine kovalente Bindung,
b) -O-,
c) -CH=CH- oder
d) -C≡C- steht,
B für
a) -(CH₂)ₘ-, worin m die ganze Zahl Null, 1, 2, 3, 4, 5 oder 6 ist,
b) -O-(CH₂)ₚ, worin p eine ganze Zahl von 1 bis 5 bedeutet, oder
c) -CH=CH- steht und
X für -CH=CH-, Sauerstoffatom oder Schwefelatom steht,
mit Ausnahme der Verbindungen 2-(Biphenyl-4-sulfonylamino)-5-(2,3-dihydro-indol-1-yl)-5-oxo-pentancarbonsäure und 5-(2,3-Dihydroindol-1-yl)-5-oxo-2-(4'-pyrrolidin-1-ylbiphenyl-4-sulfonylamino)-pentancarbonsäure und mit Ausnahme des Falles, dass in der Teilformel IIb r die ganze Zahl Null und z eine kovalente Bindung darstellen.

2. Verbindung der Formel I gemäß Anspruch 1, **dadurch gekennzeichnet, daß**
R¹ für
1. Phenyl oder
2. Phenyl, welches einfach substituiert ist durch
2.1. (C₁-C₆)-Alkyl-, worin Alkyl gerade, cyclisch oder verzweigt ist,
2.2. -OH,
2.3. (C₁-C₆)-Alkyl-C(O)-O-,
2.4. (C₁-C₆)-Alkyl-O-,
2.5. (C₁-C₆)-Alkyl-O-(C₁-C₄)-Alkyl-O-,
2.6. Halogen,
2.7. -CF₃ oder
2.8. R⁴-(R⁵)N- steht,
R², R⁴ und R⁵ gleich oder verschieden sind und für
1. Wasserstoffatom,
2. (C₁-C₆)-Alkyl- stehen oder
3. R⁴ und R⁵ zusammen mit der ringständigen Aminogruppe einen 4- bis 7-gliedrigen Ring bilden, worin gegebenenfalls eines der Kohlenstoffatome durch -O-, -S- oder -NH- ersetzt ist,
R³ für
1. -(C₁-C₄)-Alkyl-C(O)-N(R⁶)-R⁷, worin R⁶ und R⁷ zusammen mit dem Stickstoff, an den sie gebunden sind einen Rest der Teilformel IIa bilden, wobei in der Teilformel IIa
q die ganze Zahl Null oder 1 bedeutet,
Z ein Kohlenstoff-, Stickstoff-, Sauerstoff- oder Schwefelatom bedeutet, und
R⁸ Wasserstoffatom bedeutet oder die für R¹ oben unter 2.1 bis 2.8 beschriebene Bedeutung hat,
2. -(C₁-C₄)-Alkyl-C(O)-Y, worin
Y einen Rest der Teilformel IIc oder IId bedeutet
wobei in den Teilformeln IIc und IId
R⁸ Wasserstoffatom bedeutet oder die für R¹ oben unter 2.1 bis 2.8 beschriebene Bedeutung hat und
R⁹ für Wasserstoffatom steht, oder
3. -(C₁-C₄)-Alkyl-C(O)-N(R⁹)-(CH₂)₀-N(R⁴)-R⁵, wobei
R⁹ für Wasserstoffatom steht,
o die ganze Zahl 2 oder 3 bedeutet und
R⁴ und R⁵ gleich oder verschieden sind und für
1. Wasserstoffatom,
2. (C₁-C₆)-Alkyl- stehen oder
3. R⁴ und R⁵ zusammen mit der ringständigen Aminogruppe einen 4- bis 7-gliedrigen Ring bilden aus der Gruppe Isoxazolidin, Morpholin, Isothiazolidin, Thiomorpholin, Pyrazolidin, Imidazolidin, Piperazin, Azetidin, Pyrrolin, Pyrrolidin, Azepin, Piperidin oder Diazepin, steht,
A für eine kovalente Bindung oder -O- steht,
B für
a) -(CH₂)ₘ-, worin m die ganze Zahl Null, 1 oder 2 bedeutet, oder
b) -O-(CH₂)ₚ, worin p die ganze Zahl 1 oder 2 bedeutet, steht und
X für -CH=CH- steht.

3. Verbindung der Formel I gemäß Anspruch 1, **dadurch gekennzeichnet, daß**
R¹ für
1. Phenyl oder
2. Phenyl, welches einfach substituiert ist durch
2.1. Chlor,
2.2. Brom,
2.3. Fluor,
2.4. Pyrrolidin oder
2.5. Morpholin steht,
R² für Wasserstoffatom steht,
R³ für
1. -(C₁-C₄)-Alkyl-C(O)-N(R⁶)-R⁷, worin R⁶ und R⁷ zusammen mit dem Stickstoff, an den sie gebunden sind einen Rest der Teilformel IIa bilden, wobei in der Teilformel IIa
q die ganze Zahl Null oder 1 bedeutet,
Z ein Kohlenstoffatom bedeutet, und
R⁸ Wasserstoffatom, Chlor, Brom oder Fluor bedeutet,
2. -(C₁-C₄)-Alkyl-C(O)-Y, worin
Y einen Rest der Teilformel IIc oder IId bedeutet wobei in den Teilformeln IIc und IId R⁸ und R⁹ jeweils Wasserstoffatom bedeuten, oder
3. -(C₁-C₄)-Alkyl-C(O)-N(R⁹)-(CH₂)₀-N(R⁴)-R⁵, wobei
R⁹ für Wasserstoffatom steht,
o die ganze Zahl 2 oder 3 bedeutet und
R⁴ und R⁵ gleich oder verschieden sind und unabhängig voneinander für
1. Wasserstoffatom,
2. Phenyl oder
3. Morpholin stehen,
A für eine kovalente Bindung oder -O- steht,
B für eine kovalente Bindung steht und
X für -CH=CH- steht.

4. Verbindung der Formel I gemäß Anspruch 1 oder 2, **dadurch gekennzeichnet, daß** es eine Verbindung aus der Gruppe 2-(Biphenyl-4-sulfonylamino)-4-(naphthalen-1-yl-carbamoyl)-buttersäure, 2-(Biphenyl-4-sulfonylamino)-4-(naphthalen-2-yl-carbamoyl)-buttersäure, 2-( Biphenyl-4-sulfonylamino)-4-(2-phenylamino-ethylcarbamoyl)-buttersäure, 2-(4'-Chlorbiphenyl-4-sulfonylamino)-4-(3-morpholin-4-yl-propylcarbamoyl)-buttersäure, 4-(3-(4-(Biphenyl-4-sulfonylamino)-4-carboxy-butyrylamino)-propyl)-morpholin-4-ium-trifluoracetat, 2-(4'-Chlor-biphenyl-4-sulfonylamino)-5-(2,3-dihydroindol-1-yl)-5-oxo-pentansäure, 2-(4'-Brom-biphenyl-4-sulfonylamino)-5-(2,3-dihydroindol-1-yl)-5-oxo-pentansäure, 2-(4'-Chlor-biphenyl-4-sulfonylamino)-5-(5-fluor-2,3-dihydroindol-1-yl)-5-oxo-pentansäure, 2-(4'-Brom-biphenyl-4-sulfonylamino)-5-(5-fluor-2,3-dihydroindol-1-yl)-5-oxo-pentansäure, 5-(5-Fluor-2,3-dihydroindol-1-yl)-5-oxo-2-(4'-Pyrrolidin-1-yl-biphenyl-4-sulfonylamino)-pentansäure und 5-(5-Fluor-2,3-dihydroindol-1-yl)-2-(4'-morpholin-4-yl-biphenyl-4-sulfonylamino)-5-oxo-pentansäure ist.

5. Verbindung der Formel I gemäß Anspruch 4, **dadurch gekennzeichnet, daß** es ein 2-Amino-2-(hydroxymethyl)-1,3-propandiol-Salz, Trimethylamin-Salz, Triethylamin-Salz, Ethanolamin-Salz oder Triethanolamin-Salz ist.

6. Verfahren zur Herstellung der Verbindung der Formel I, gemäß einem oder mehreren der Ansprüche 1 bis 5, **dadurch gekennzeichnet, daß** man
a) eine Aminocarbonsäure der Formel II, 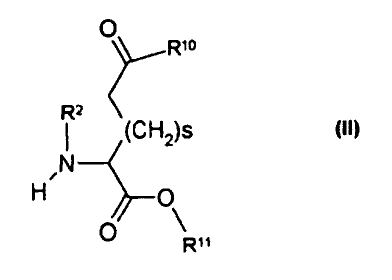 worin R¹¹ ein Wasserstoffatom oder eine in der Peptidchemie übliche Ester-Schutzgruppe bedeutet, s die ganze Zahl Null, 1, 2 oder 3 bedeutet, R¹⁰ den Rest -OR¹² bedeutet, worin R¹² eine in Gegenwart von R¹¹ spaltbare Ester-Schutzgruppe oder ein Wasserstoffatom ist, oder R¹⁰ einen Rest -N(R⁶)-R⁷ bedeutet, worin R⁶ und R⁷ wie in Formel I gemäß Anspruch 1 definiert sind, mit einem Sulfonsäurederivat der Formel III, 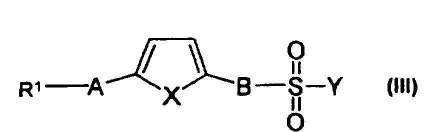 wobei R¹, X, A und B wie in Formel I definiert sind und Y ein Halogenatom, Imidazolyl oder OR¹³ bedeutet, worin R¹³ Wasserstoffatom, (C₁-C₆)-Alkyl, Phenyl oder Benzyl, welches gegebenenfalls substituiert ist, darstellt,
in Gegenwart einer Base oder gegebenenfalls eines wasserentziehenden Mittels zu einer Verbindung der Formel IV, 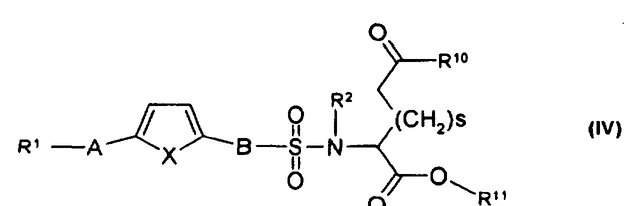 worin R¹, A, X, B, R², s, R¹⁰ und R¹¹ die oben genannte Bedeutung haben, umsetzt und anschließend eine eventuell im Rest R¹⁰ mitgeführte Schutzgruppe R¹² in Gegenwart der Schutzgruppe R¹¹ abspaltet, und danach den Rest -N(R⁶)-R⁷ nach entsprechender, aus der Peptidchemie bekannter Aktivierung der Carboxylgruppe einführt, und abschließend die mitgeführte Schutzgruppe R¹¹ abspaltet und die Verbindung der Formel I erhält, oder
b) ein Aminosäureanhydrid der Formel V, 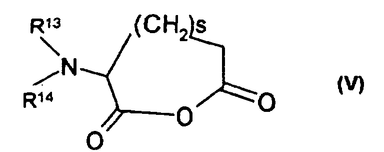 worin R¹³ ein Wasserstoffatom und R¹⁴ eine in der Peptidchemie bekannte N-Schutzgruppe darstellt, oder R¹³ und R¹⁴ eine cydische N-Schutz-gruppe, z.B Phthalimido, bedeuten und s wie oben unter Verfahren a) definiert ist, mit einem primären oder sekundären Rest -N(R⁶)-R⁷ im Sinne einer Ringöffnung zu einem Zwischenprodukt der Formel VI umsetzt, 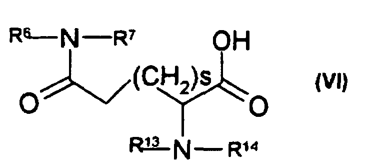 worin R¹³, R¹⁴, s, R⁶ und R⁷ die obengenannte Bedeutung haben, wobei diese Ringöffnung in Abhängigkeit von Schutzgruppe und Reaktionsbe-dingungen in der Regel regioselektiv das in Formel VI gezeigte isomere liefert, das beim Auftreten von Regioisomerengemischen durch Kristallisation oder Chromatographie weiter angereichert werden kann, anschließend die mitgeführte Schutzgruppe R¹³ und/oder R¹⁴ unter Feisetzung des Amines spaltet, und danach wie unter a) beschrieben, mit einem Sulfonsäurederivat der Formel III eine N-Sulfonierung durchführt, die zu einem Produkt der Formel I führt, oder
c) eine nach den Verfahren a) oder b) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
d) die nach den Verfahren a), b) oder c) hergestellte Verbindung der Formel I entweder in freier Form isoliert oder im Falle des Vorliegens von sauren oder basischen Gruppen in physiologisch verträgliche Salze umwandelt.

7. Arzneimittel, **gekennzeichnet durch** einen wirksamen Gehalt an einer Verbindung der Formel I gemäß einem oder mehreren der Ansprüche 1 bis 5 zusammen mit einem pharmazeutisch geeigneten und physiologisch verträglichen Trägerstoff, Zusatzstoff und/oder anderen Wirk- und Hilfsstoffen.

8. Verwendung von mindestens einer Verbindung der Formel I gemäß einem oder mehreren der Ansprüche 1 bis 5, zur Herstellung von Arzneimitteln zur Prophylaxe und Therapie von Erkrankungen, an deren Verlauf Matrix-abbauende Metalloproteinasen beteiligt sind.

9. Verwendung von mindestens einer Verbindung der Formel I gemäß einem oder mehreren der Ansprüche 1 bis 5 zur Herstellung von Arzneimitteln zur Prophylaxe und Therapie von degenerative Gelenkerkrankungen wie Osteoarthrosen, rheumatoide Arthritis, Spondylosen, Knorpelschwund nach Gelenktrauma oder längerer Gelenksruhigstellung, nach Meniskus- oder Patellaverletzungen oder Bänder-rissen oder Erkrankungen des Bindegewebes wie Kollagenosen, Periodontal-erkrankungen, Wundheilungsstörungen und chronische Erkrankungen des Bewegungsapparates wie entzündliche, immunologisch oder stoffwechsel-bedingte akute und chronische Arthritiden, Arthropathien, Myalgien und Störungen des Knochenstoffwechsels wie Osteoporose, oder Gefäß-krankheiten, z.B. Blutgefäßverschlüssen, atherosklerotischen Plaques oder Aneurysmen, bei drohender Ruptur, oder bei Stenosen jedweder Patho-genese oder zur Behandlung von Entzündungen, einschließlich Wunden und Geschwüren, insbesondere auch der Haut, Krebserkrankungen, zur Blockierung oder Eindämmung der Metastasenbildung und Metastasen-ausbreitung, bei Mammakarzinom oder bei Kachexie, Anorexie, septischem Schock, Periodontose oder Periodontitis.

10. Verfahren zur Herstellung eines Arzneimittels, **dadurch gekennzeichnet, daß** man mindestens eine Verbindung der Formel I gemäß einem oder mehreren der Ansprüche 1 bis 5 mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

## Claims

1. A compound of the formula 1 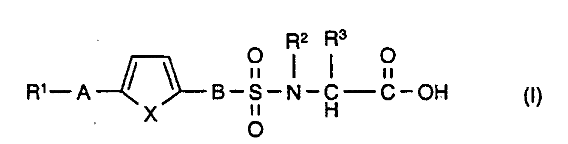 and/or a stereoisomeric form of the compound of the formula I and/or a physiologically tolerable salt of the compound of the formula I, where
R¹ is
1. phenyl,
2. phenyl, which is mono- or disubstituted by
2.1. (C₁-C₆)-alkyl, which is linear, cyclic or branched,
2.2. hydroxyl,
2.3. (C₁-C₆)-alkyl-C(O)-O-,
2.4. (C₁-C₆)-alkyl-O-,
2.5. (C₁-C₆)-alkyl-O-(C₁-C₄)-alkyl-O-,
2.6. halogen,
2.7. -CF₃,
2.8. -CN,
2.9. -NO₂,
2.10. HO-C(O)-,
2.11. (C₁-C₆)-alkyl-O-C(O)-,
2.12. methylenedioxo,
2.13. R⁴-(R⁵ )N-C(O)-,
2.14. R⁴-(R⁵ )N-, or
2.15. the radical of a heterocycle from the following group: isoxazolidine, morpholine, isothiazolidine, thiomorpholine, pyrazolidine, imidazolidine, piperazine, azetidine, pyrrole, pyrroline, pyrrolidine, pyridine, azepine, piperidine, oxazole, isoxazole, imidazole, pyrazole, thiazole, isothiazole, diazepine, thiomorpholine, pyrimidine and pyrazine, which is unsubstituted or substituted as described for R¹ under 2.1 to 2.14, or
3. a heteroaromatic from the following group 3.1. to 3.15., which is unsubstituted or substituted as described for R¹ under 2.1 to 2.14,
3.1. pyrrole,
3.2. pyrazole,
3.3. imidazole,
3.4. triazole,
3.5. thiophene,
3.6. thiazole,
3.7. oxazole,
3.8. isoxazole,
3.9. pyridine,
3.10. pyrimidine,
3.11. indole,
3.12. benzothiophene,
3.13. benzimidazole,
3.14. benzoxazole or
3.15. benzothiazole,
R², R⁴ and R⁵ are identical or different and are
1. a hydrogen atom,
2. (C₁-C₆)-alkyl-,
3. HO-C(O)-(C₁-C₆)-alkyl-,
4. phenyl-(CH₂)ₙ-, in which phenyl is unsubstituted or mono- or disubstituted as described for R¹ under 2.1. to 2.14. and n is the integer zero, 1 or 2, or
5. picolyl or
6. R⁴ and R⁵, together with the ring amino group, form a 4- to 7-membered ring in which, if appropriate, one of the carbon atoms is replaced by -O-, -S- or -NH-,
R³ is
1. -(C₁-C₄)-alkyl-C(O)-N(R⁶)-R⁷, in which R⁶ and R⁷ together with the nitrogen to which they are bonded form a radical of the subformula IIa, IIb or IIe 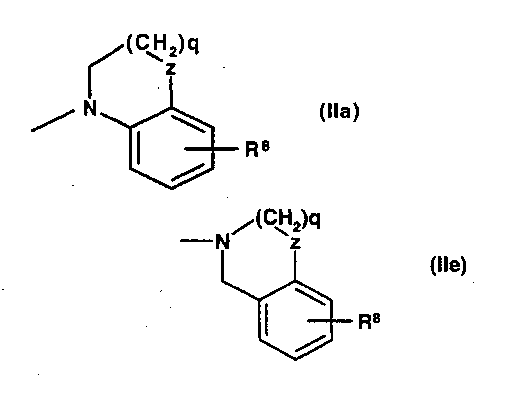 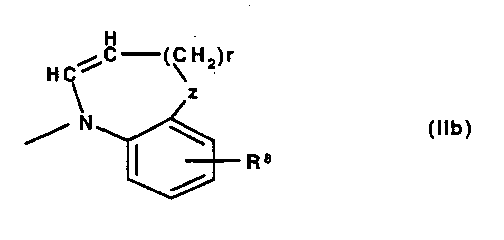 where in the subformulae IIa, IIb and IIe
q is the integer zero, 1 or 2 and
r is the integer zero or 1,
z is a carbon, nitrogen, oxygen or sulfur atom or a covalent bond, and
R⁸ is a hydrogen atom or has the meaning described for R¹ above under 2.1 to 2.14,
2. -(C₁-C₄)-alkyl-C(O)-Y,
in which Y is a radical of the subformula IIc or IId 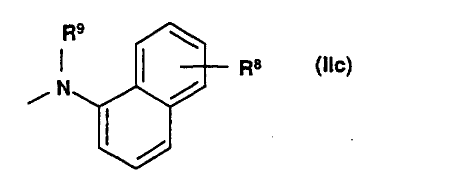 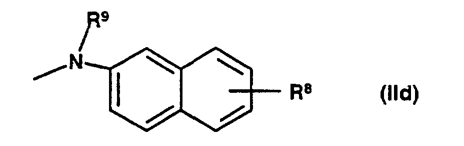 where in the subformulae IIc and IId
R⁸ is a hydrogen atom or has the meaning described for R¹ above under 2.1 to 2.14 and
R⁹ is
2.1 a hydrogen atom
2.2 (C₁-C₆)-alkyl-,
2.3 HO-C(O)-(C₁-C₆)-alkyl-,
2.4 phenyl-(CH₂)ₙ-, in which phenyl is unsubstituted or mono- or disubstituted as described for R¹ under 2.1 to 2.14 and n is the integer zero, 1 or 2, or
2.5 picolyl, or
3. -(C₁-C₄)-alkyl-C(O)-N(R⁹)-(CH₂)ₒ-N(R⁴)-R⁵ ,
where
R⁹ has the abovementioned meaning,
o is the integer 2, 3, 4 or 5 and
R⁴ and R⁵ together with the ring amino group form a 4- to 7-membered ring from the group consisting of isoxazolidine, morpholine, isothiazolidine, thiomorpholine, pyrazolidine, imidazolidine, piperazine, acetidine, pyrroline, pyrrolidine, azepine, piperidine and diazepine,
A is
a) a covalent bond,
b) -O-,
c) -CH=CH- or
d) -C≡C-,
B is
a) -(CH₂)ₘ-, in which m is the integer zero, 1, 2, 3, 4, 5 or 6,
b) -O-(CH₂)ₚ, in which p is an integer from 1 to 5, or
c) -CH=CH- and
X is -CH=CH-, an oxygen atom or a sulfur atom, with the exception of the compounds 2-(biphenyl-4-sulfonylamino)-5-(2,3-dihydroindol-1-yl)-5-oxo-pentanecarboxylic acid and 5-(2,3-dihydroindol-1-yl)-5-oxo-2-(4'-pyrrolidin-1-ylbiphenyl-4-sulfonylamino)pentanoic acid and with the exception of the case in which the subformula IIb r is the integer zero and z is a covalent bond.

2. A compound of the formula I as claimed in claim 1, wherein
R¹ is
1. phenyl or
2. phenyl, which is monosubstituted by
2.1. (C₁-C₆)-alkyl-, in which alkyl is linear, cyclic or branched,
2.2. -OH,
2.3. (C₁-C₆)-alkyl-C(O)-O-,
2.4. (C₁-C₆)-alkyl-O-,
2.5. (C₁-C₆)-alkyl-O-(C₁-C₄)-alkyl-O-,
2.6. halogen,
2.7. -CF₃ or
2.8. R⁴-(R⁵)N-,
R², R⁴ and R⁵ are identical or different and are
1. a hydrogen atom,
2. (C₁-C₆)-alkyl- or
3. R⁴ and R⁵ together with the ring amino group form a 4- to 7-membered ring, in which, if appropriate, one of the carbon atoms is replaced by -O-, -S- or -NH-,
R³ is
1. -(C₁-C₄)-alkyl-C(O)-N(R⁶)-R⁷, in which R⁶ and R⁷ together with the nitrogen to which they are bonded form a radical of the subformula IIa, where in the subformula IIa
q is the integer zero or 1,
Z is a carbon, nitrogen, oxygen or sulfur atom, and
R⁸ is a hydrogen atom or has the meaning described for R¹ above under 2.1 to 2.8,
2. -(C₁-C₄)-alkyl-C(O)-Y, in which
Y is a radical of the subformula IIc or IId
where in the subformulae IIc and IId
R⁸ is a hydrogen atom or has the meaning described for R¹ above under 2.1 to 2.8 and
R⁹ is a hydrogen atom, or
3. -(C₁-C₄)-alkyl-C(O)-N(R⁹)-(CH₂)ₒ-N(R⁴)-R⁵, where
R⁹ is a hydrogen atom,
o is the integer 2 or 3 and
R⁴ and R⁵ are identical or different and are
1. a hydrogen atom,
2. (C₁-C₆)-alkyl- or
3. R⁴ and R⁵ together with the ring amino group form a 4- to 7-membered ring from the group consisting of isoxazolidine, morpholine, isothiazolidine, thiomorpholine, pyrazolidine, imidazolidine, piperazine, acetidine, pyrroline, pyrrolidine, azepine, piperidine and diazepine,
A is a covalent bond or -O-,
B is
a) -(CH₂)ₘ-, in which m is the integer zero, 1 or 2, or
b) -O-(CH₂)ₚ, in which p is the integer 1 or 2, and
X is -CH=CH-.

3. A compound of the formula I as claimed in claim 1, wherein
R¹ is
1. phenyl or
2. phenyl, which is monosubstituted by
2.1. chlorine,
2.2. bromine,
2.3. fluorine,
2.4. pyrrolidine or
2.5. morpholine,
R² isa hydrogen atom,
R³ is
1. -(C₁-C₄)-alkyl-C(O)-N(R⁶)-R⁷, in which R⁶ and R⁷ together with the nitrogen to which they are bonded form a radical of the subformula IIa, where in the subformula IIa
q is the integer zero or 1,
Z is a carbon atom, and
R⁸ is a hydrogen atom, chlorine, bromine or fluorine,
2. -(C₁-C₄)-alkyl-C(O)-Y, in which
Y is a radical of the subformula IIc or IId
where in the subformulae IIc and IId
R⁸ and R⁹ are each a hydrogen atom, or
3. -(C₁-C₄)-alkyl-C(O)-N(R⁹)-(CH₂)ₒ-N(R⁴)-R⁵, where
R⁹ is a hydrogen atom,
o is the integer 2 or 3 and
R⁴ and R⁵ are identical or different and independently of one another are
1. a hydrogen atom,
2. phenyl or
3. morpholine,
A is a covalent bond or -O-,
B is a covalent bond and
X is -CH=CH-.

4. A compound of the formula I as claimed in claim 1 or 2, which is a compound from the group consisting of 2-(biphenyl-4-sulfonylamino)-4-(naphthalen-1-ylcarbamoyl)butyric acid, 2-(biphenyl-4-sulfonylamino)-4-(naphthalen-2-yl-carbamoyl)butyric acid, 2-(biphenyl-4-sulfonylamino)-4-(2-phenylaminoethylcarbamoyl)butyric acid, 2-(4'-chlorobiphenyl-4-sulfonylamino)-4-(3-morpholin-4-yl-propylcarbamoyl)butyric acid, 4-(3-(4-(biphenyl-4-sulfonylamino)-4-carboxybutyrylamino)propyl)morpholin-4-ium trifluoroacetate, 2-(4'-chlorobiphenyl-4-sulfonylamino)-5-(2,3-dihydroindol-1-yl)-5-oxopentanoic acid, 2-(4'-bromobiphenyl-4-sulfonylamino)-5-(2,3-dihydroindol-1-yl)-5-oxopentanoic acid, 2-(4'-chloro-biphenyl-4-sulfonylamino)-5-(5-fluoro-2,3-dihydroindol-1-yl)-5-oxopentanoic acid, 2-(4'-bromobiphenyl-4-sulfonylamino)-5-(5-fluoro-2,3-dihydroindol-1-yl)-5-oxopentanoic acid, 5-(5-fluoro-2,3-dihydroindol-1-yl)-5-oxo-2-(4'-pyrrolidin-1-yl-biphenyl-4-sulfonylamino)pentanoic acid and 5-(5-fluoro-2,3-dihydroindol-1-yl)-2-(4'-morpholin-4-yl-biphenyl-4-sulfonylamino)-5-oxopentanoic acid.

5. A compound of the formula I as claimed in claim 4, which is a 2-amino-2-hydroxymethyl-1,3-propanediol salt, trimethylamine salt, triethylamine salt, ethanolamine salt or triethanolamine salt.

6. A process for the preparation of the compound of the formula I, as claimed in one or more of claims 1 to 5, which comprises
a) reacting an aminocarboxylic acid of the formula II 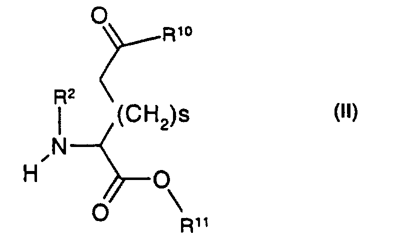 in which R¹¹ is a hydrogen atom or an ester protective group which is customary in peptide chemistry, s is the integer zero, 1, 2 or 3, R¹⁰ is the radical -OR¹², in which R¹² is an ester protective group, which can be cleaved in the presence of R¹¹, or a hydrogen atom, or R¹⁰ is a radical -N(R⁶)-R⁷, in which R⁶ and R⁷ are as defined in formula I as claimed in claim 1, with a sulfonic acid derivative of the formula III 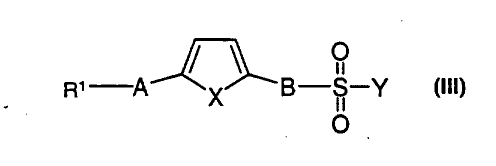 where R¹, X, A and B are as defined in formula I and Y is a halogen atom, imidazolyl or OR¹³, in which R¹³ is a hydrogen atom, (C₁-C₆)-alkyl, phenyl or benzyl, which is optionally substituted,
in the presence of a base or, if appropriate, of a dehydrating agent to give a compound of the formula IV 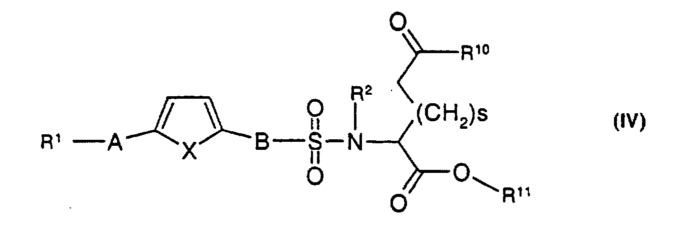 in which R¹, A, X, B, R², s, R¹⁰ and R¹¹ have the abovementioned meaning, and then removing a protective group R¹² possibly included in the radical R¹⁰ in the presence of the protective group R¹¹, and then introducing the radical -N(R⁶)-R⁷ by appropriate activation, known from peptide chemistry, of the carboxyl group, and subsequently removing the included protective group R¹¹ and obtaining the compound of the formula I, or
b) reacting an amino acid anhydride of the formula V 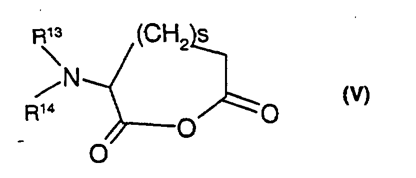 in which R¹³ is a hydrogen atom and R¹⁴ is an N-protective group known from peptide chemistry, or R¹³ and R¹⁴ are a cyclic N-protective group, e.g. phthalimido and s is as defined above under process a),
with a primary or secondary radical -N(R⁶)-R⁷ in the sense of a ring opening to give an intermediate of the formula VI 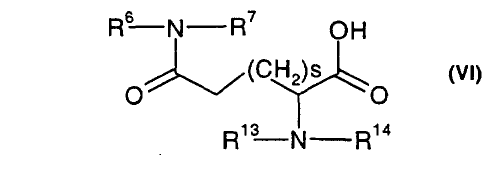 in which R¹³, R¹⁴, s, R⁶ and R⁷ have the abovementioned meaning, where this ring opening, depending on the protective group and reaction conditions as a rule regioselectively yields the isomer shown in formula VI, which, if regioisomer mixtures occur, can be further enriched by crystallization or chromatography, then cleaving the included protective group R¹³ and/or R¹⁴ with release of the amine, and then carrying out, as described under a), an N-sulfonation with a sulfonic acid derivative of the formula III which leads to a product of the formula I, or
c) separating a compound of the formula I prepared according to process a) or b), which on account of its chemical structure occurs in enantiomeric forms, into the pure enantiomers by salt formation with enantiomerically pure acids or bases, chromatography on chiral stationary phases or derivatization by means of chiral enantiomerically pure compounds such as amino acids, separation of the diastereomers thus obtained, and removal of the chiral auxiliary groups, or
d)isolating the compound of the formula I prepared according to process a), b) or c) in free form or, if acidic or basic groups are present, converting it into physiologically tolerable salts.

7. A pharmaceutical, which contains an efficacious amount of a compound of the formula I as claimed in one or more of. claims 1 to 5 together with a pharmaceutically suitable and physiologically tolerable excipient, additive and/or other active compounds and auxiliaries.

8. The use of at least one compound of the formula I as claimed in one or more of claims 1 to 5 for the production of pharmaceuticals for the prophylaxis and therapy of disorders in whose course matrix-degrading metalloproteinases are involved.

9. The use of at least one compound of the formula I as claimed in one or more of claims 1 to 5 for the production of pharmaceuticals for the prophylaxis and therapy of degenerative joint disorders such as osteoarthroses, rheumatoid arthritis, spondyloses, chondrolysis after joint trauma or relatively long immobilization of the joint, after meniscus or patella injuries or tearing of the ligaments or disorders of the connective tissue such as collagenoses, periodontal disorders, wound healing disorders and chronic disorders of the locomotory apparatus such as inflammatory, immunologically or metabolically caused acute and chronic arthritis, arthropathies, myalgias and disorders of the bone metabolism such as osteoporosis, or vascular diseases, e.g. blood vessel occlusions, atherosclerotic plaques or aneurisms, in threatening rupture, or in stenoses of any pathogenesis or for the treatment of inflammations, including wounds and ulcers, in particular also of the skin, carcinomatous disorders, for blocking or checking the formation and spread of metastases, in carcinoma of the breast or in cachexia, anorexia, septic shock, periodontosis or periodontitis.

10. A process for the production of a pharmaceutical, which comprises bringing at least one compound of the formula I as claimed in one or more of claims 1 to 5 into a suitable administration form using a pharmaceutically suitable and physiologically tolerable vehicle and, if appropriate, further suitable active compounds, additives or auxiliaries.

## Revendications

1. Composé de formule I 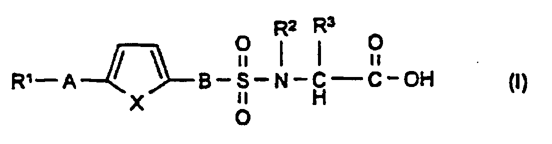 et/ou forme stéréo-isomère du composé de formule I et/ou un physiologiquement acceptable du composé de formule I, où
R¹ représente:
1. un groupe phényle,
2. un groupe phényle substitué une ou deux fois par
2.1. alkyle en C₁-C₆ linéaire, cyclique ou ramifié,
2.2. hydroxy,
2.3. (alkyl en C₁-C₆)-C(O)-O-
2.4. (alkyl en C₁-C₆)-O-,
2.5. (alkyl en C₁-C₆)-O-(alkyl en C₁-C₄)-O-,
2.6. halogéno,
2.7. -CF₃,
2.8. -CN,
2.9. -NO₂,
2.10. HO-C(O)-,
2.11. (alkyl en C₁-C₆)-O-C(O)-,
2.12. méthylènedioxo,
2.13. R⁴-(R⁵)N-C(O)-,
2.14. R⁴-(R⁵)N- ou
2.15. un reste d'un hétérocycle du groupe suivant: l'isoxazolidine, la morpholine, l'isothiazolidine, la thiomorpholine, la pyrazolidine, l'imidazolidine, la pipérazine, l'azétidine, le pyrrole, la pyrroline, la pyrrolidine, la pyridine, l'azépine, la pipéridine, l'oxazole, l'isoxazole, l'imidazole, le pyrazole, le thiazole, l'isothiazole, la diazépine, la thiomorpholine, la pyrimidine et la pyrazine, non substitué ou substitué de la manière décrite pour R¹ dans 2.1 à 2.14, ou
3. un groupe hétéroaromatique du groupe constitué par 3.1 à 3.15 ci-dessus, non substitué ou substitué de la manière décrite pour R¹ dans 2.1 à 2.14:
3.1. le pyrrole,
3.2. le pyrazole,
3.3. l'imidazole,
3.4. le triazole,
3.5. le thiophène,
3.6. le thiazole,
3.7. l'oxazole,
3.8. l'isoxazole,
3.9. la pyridine,
3.10. la pyrimidine,
3.11. l'indole,
3.12. le benzothiophène,
3.13. le benzimidazole,
3.14. le benzoxazole, ou
3.15. le benzothiazole,
R², R⁴ et R⁵ sont identiques ou différents et représentent:
1. un atome d'hydrogène,
2. un groupe alkyle en C₁-C₆,
3. un groupe HO-C(O)-(alkyle en C₁-C₆),
4. un groupe phényle-(CH₂)ₙ, dans lequel le groupe phényle est non substitué ou substitué une ou deux fois de la manière décrite pour R' dans 2.1 à 2.14, et n représente le nombre entier 0, 1 ou 2, ou
5. un groupe picolyle, ou
6. R⁴ et R⁵ forment ensemble, avec le groupe amino cyclique, un cycle de 4 à 7 chaînons, dans lequel l'un des atomes de carbone est éventuellement remplacé par -O-, -S- ou -NH-,
R³ représente:
1. un groupe -(alkyl en C₁-C₄)-C(O)-N(R⁶)-R⁷, dans lequel R⁶ et R⁷ forment ensemble, avec l'atome d'azote auquel ils sont liés, un reste ayant la formule partielle IIa, IIb ou IIe 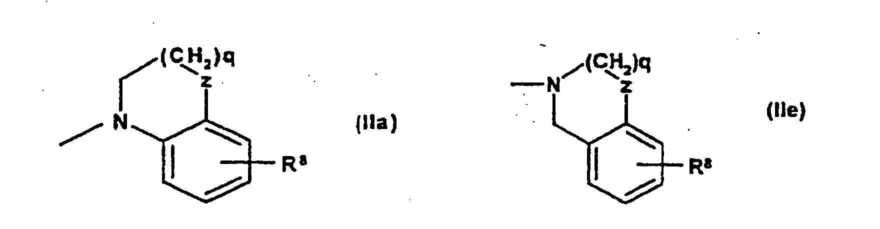 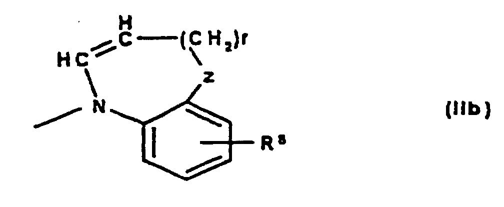 où, dans les formules partielles IIa, IIb et IIe
q représente le nombre entier 0, 1 ou 2 et
r représente le nombre entier 0 ou 1,
Z représente un atome de carbone, d'azote, d'oxygène ou de soufre ou une liaison covalente, et
R⁸ représente un atome d'hydrogène ou a la signification décrite pour R¹ ci-dessus dans 2.1 à 2.14,
2. un groupe -(alkyl en C₁-C₄)-C(O)-Y, dans lequel Y représente un reste ayant la formule partielle IIc ou IId 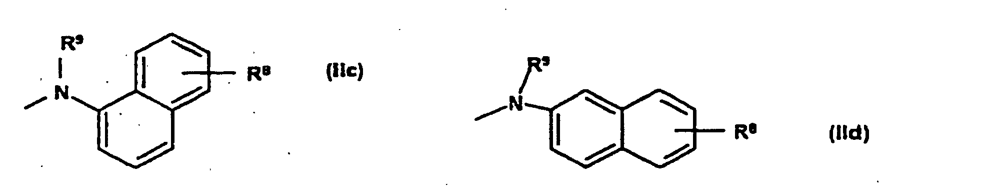 où, dans les formules partielles IIc et IId:
R⁸ représente un atome d'hydrogène ou a la signification décrite pour R¹ ci-dessus dans 2.1 à 2.14,
R⁹ représente
2.1 un atome d'hydrogène,
2.2 un groupe alkyle en C₁-C₆,
2.3 un groupe HO-C(O)-(alkyle en C₁-C₆),
2.4 un groupe phényle-(CH₂)ₙ, dans lequel le groupe phényle est non substitué ou substitué une ou deux fois de la manière décrite pour R¹ dans 2.1 à 2.14, et n représente le nombre entier 0, 1 ou 2, ou
2.5 un groupe picolyle, ou
3. un groupe -(alkyl en C₁-C₄)-C(O)-N(R⁹)-(CH₂)ₒ-N(R⁴)-R⁵ dans lequel
R⁹ a la signification donnée ci-dessus,
o est le nombre entier 2, 3, 4 ou 5, et
R⁴ et R⁵ représentent ensemble, avec le groupe amino cyclique, un cycle de 4 à 7 chaînons du groupe constitué par l'isoxazolidine, la morpholine, l'isothiazolidine, la thiomorpholine, la pyrazolidine, l'imidazolidine, la pipérazine, l'azétidine, la pyrroline, la pyrrolidine, l'azépine, la pipéridine ou la diazépine,
A représente
a) une liaison covalente,
b) -O-,
c) -CH=CH- ou
d) -C≡C -
B représente
a) -(CH₂)ₘ où m est le nombre entier 0, 1, 2, 3, 4, 5 ou 6,
b) -O-(CH₂)ₚ où p est un nombre entier de 1 à 5, ou
c) -CH=CH-, et
X représente -CH=CH-, un atome d'oxygène ou un atome de soufre, à l'exception des composés acide 2-(biphényl-4-sulfonylamino)-5-(2,3-dihydroindol-1-yl)-5-oxopentanoïque et acide 5-(2,3-dihydroindol-1-yl)-5-oxo-2-(4'-pyrrolidin-1-ylbiphényl-4-sulfonylamino)pentanoïque, et à l'exception du cas dans lequel, dans la formule partielle IIb, r est le nombre entier 0 et Z représente une liaison covalente.

2. Composé de formule I selon la revendication 1, **caractérisé en ce que**
R¹ représente:
1. un groupe phényle, ou
2. un groupe phényle substitué une fois par
2.1. alkyle en C₁-C₆, le groupe alkyle étant linéaire, cyclique ou ramifié,
2.2. OH,
2.3. (alkyl en C₁-C₆)-C(O)-O-
2.4. (alkyl en C₁-C₆)-O-,
2.5. (alkyl en C₁-C₆)-O-(alkyl en C₁-C₄)-O-,
2.6. halogéno,
2.7. -CF₃, ou
2.8. R⁴-(R⁵)N-,
R², R⁴ et R⁵ sont identiques ou différents et représentent:
1. un atome d'hydrogène,
2. un groupe alkyle en C₁-C₆, ou
3. R⁴ et R⁵ forment ensemble, avec le groupe amino cyclique, un cycle de 4 à 7 chaînons, dans lequel l'un des atomes de carbone est éventuellement remplacé par -O-, -S- ou -NH-,
R³ représente:
1. un groupe -(alkyl en C₁-C₄)-C(O)-N(R⁶)-R⁷, dans lequel R⁶ et R⁷ forment ensemble, avec l'atome d'azote auquel ils sont liés, un reste ayant la formule partielle IIa,
où, dans la formule partielle IIa,
q représente un nombre entier 0 ou 1,
Z représente un atome de carbone, d'azote, d'oxygène ou de soufre, et
R⁸ représente un atome d'hydrogène ou a la signification décrite pour R¹ ci-dessus dans 2.1 à 2.8,
2. un groupe -(alkyl en C₁-C₄)-C(O)-Y, dans lequel Y représente un reste ayant la formule partielle IIc ou IId
où, dans les formules partielles IIc et IId:
R⁸ représente un atome d'hydrogène ou a la signification décrite pour R¹ ci-dessus dans 2.1 à 2.8,
R⁹ représente un atome d'hydrogène, ou
3. un groupe -(alkyl en C₁-C₄)-C(O)-N(R⁹)-(CH₂)ₒ-N(R⁴)-R⁵ dans lequel
R⁹ représente un atome d'hydrogène,
o est le nombre entier 2 ou 3, et
R⁴ et R⁵ sont identiques ou différents et représentent
1. un atome d'hydrogène,
2. un groupe alkyle en C₁-C₆, ou
3. R⁴ et R⁵ représentent ensemble, avec le groupe amino cyclique, un cycle de 4 à 7 chaînons du groupe constitué par l'isoxazolidine, la morpholine, l'isothiazolidine, la thiomorpholine, la pyrazolidine, l'imidazolidine, la pipérazine, l'azétidine, la pyrroline, la pyrrolidine, l'azépine, la pipéridine ou la diazépine,
A représente une liaison covalente ou -O-,
B représente
a) -(CH₂)ₘ où m est le nombre entier 0, 1 ou 2,
b) -O-(CH₂)ₚ où p est le nombre entier 1 ou 2, et
X représente le groupe -CH=CH-.

3. Composé de formule I selon la revendication 1, **caractérisé en ce que**
R¹ représente:
1. un groupe phényle, ou
2. un groupe phényle substitué une fois par
2.1. chloro,
2.2. bromo,
2.3. fluoro
2.4. pyrrolidine ou
2.5. morpholine,
R² représente un atome d'hydrogène,
R³ représente:
1. un groupe -(alkyl en C₁-C₄)-C(O)-N(R⁶)-R⁷, dans lequel R⁶ et R⁷ forment ensemble, avec l'atome d'azote auquel ils sont liés, un reste ayant la formule partielle IIa,
où, dans la formule partielle IIa,
q représente le nombre entier 0 ou 1,
Z représente un atome de carbone et
R⁸ représente un atome d'hydrogène, de chlore, de brome ou de fluor,
2. un groupe -(alkyl en C₁-C₄)-C(O)-Y, dans lequel Y représente un reste ayant la formule partielle IIc ou IId, où, dans les formules partielles IIc et IId, R⁸ et R⁹ représentent chacun un atome d'hydrogène, ou
3. un groupe -(alkyl en C₁-C₄)-C(O)-N(R⁹)-(CH₂)ₒ-N(R⁴)-R⁵ dans lequel
R⁹ représente un atome d'hydrogène,
o est le nombre entier 2 ou 3, et
R⁴ et R⁵ sont identiques ou différents et représentent, indépendamment l'un de l'autre
1. un atome d'hydrogène,
2. un groupe phényle, ou
3. un groupe morpholine,
A représente une liaison covalente ou -O-,
B représente une liaison covalente, et
X représente le groupe -CH=CH-.

4. Composé de formule I selon la revendication 1 ou 2, **caractérisé en ce qu'**il s'agit d'un composé du groupe constitué par l'acide 2-(biphényl-4-sulfonylamino)-4-(naphtalén-1-ylcarbamoyl)butyrique, l'acide 2-(biphényl-4-sulfonylamino)-4-(naphtalén-2-ylcarbamoyl)butyrique, l'acide 2-(biphényl-4-sulfonylamino)-4-(2-phénylaminoéthylcarbamoyl)butyrique, l'acide 2-(4'-chlorobiphényl-4-sulfonylamino)-4-(3-morpholin-4-ylpropylcarbamoyl)butyrique, le trifluoroacétate de 4-(3-(4-(biphényl-4-sulfonylamino)-4-carboxybutyrylamino)propyl)morpholin-4-ium, l'acide 2-(4'-chlorobiphényl-4-sulfonylamino)-5-(2,3-dihydroindol-1-yl)-5-oxopentanoïque, l'acide 2-(4'-bromobiphényl-4-sulfonylamino)-5-(2,3-dihydroindol-1-yl)-5-oxopentanoïque, l'acide 2-(4'-chlorobiphényl-4-sulfonylamino)-5-(5-fluoro-2,3-dihydroindol-1-yl)-5-oxopentanoïque, l'acide 2-(4'-bromobiphényl-4-sulfonylamino)-5-(5-fluoro-2,3-dihydroindol-1-yl)-5-oxopentanoïque, l'acide 5-(5-fluoro-2,3-dihydroindol-1-yl)-5-oxo-2-(4'-pyrrolidin-1-ylbiphényl-4-sulfonylamino)pentanoïque et l'acide 5-(5-fluoro-2,3-dihydroindol-1-yl)-2-(4'-morpholin-4-ylbiphényl-4-sulfonylamino)-5-oxopentanoïque.

5. Composé de formule I selon la revendication 4, **caractérisé en ce qu'**il s'agit d'un sel de 2-amino-(2-hydroxyméthyl)-1,3-propanediol, d'un sel de triméthylamine, d'un sel de triéthylamine, d'un sel d'éthanolamine ou d'un sel de triéthanolamine.

6. Procédé de préparation du composé de formule I selon l'une ou plusieurs des revendications 1 à 5, **caractérisé en ce que**
a) on fait réagir un acide aminocarboxylique de formule II 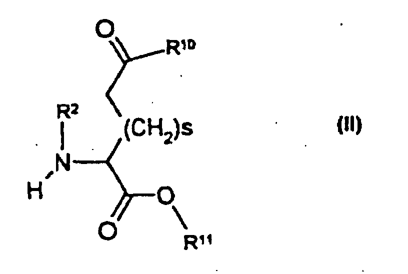 dans laquelle R¹¹ représente un atome d'hydrogène ou un groupe protecteur ester classique dans la chimie des peptides, s représente le nombre entier 0, 1, 2 ou 3, R¹⁰ représente le reste -OR¹² dans lequel R¹² représente un atome d'hydrogène ou un groupe protecteur ester pouvant se dissocier en présence de R¹¹, ou R¹⁰ représente un reste -N(R⁶)-R⁷ dans lequel R⁶ et R⁷ ont la définition donnée dans la formule I selon la revendication 1,
avec un dérivé d'acide sulfonique de formule III 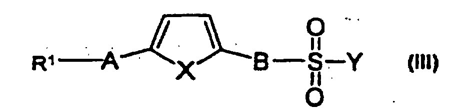 dans laquelle R¹, X, A et B ont la même définition que dans la formule I, et Y représente un atome d'halogène ou un groupe imidazolyle ou OR¹³, où R¹³ représente un atome d'hydrogène ou un groupe alkyle en C₁-C₆, phényle ou benzyle éventuellement substitué,
en présence d'une base ou éventuellement d'un agent déshydratant, pour obtenir un composé de formule IV 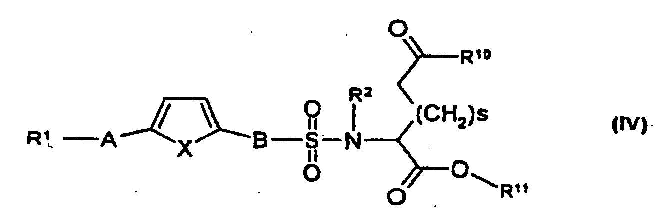 dans laquelle R¹, A, X, B, R², s, R¹⁰ et R¹¹ ont la signification indiquée ci-dessus, on sépare ensuite le groupe protecteur R¹² éventuellement introduit dans le reste R¹⁰ en présence du groupe protecteur R¹¹, puis on introduit le reste -N(R⁶)-R⁷ par activation du groupe carboxyle de manière connue dans la chimie des peptides, on sépare ensuite le groupe protecteur R¹¹ introduit et on obtient le composé de formule I, ou
b) on fait réagir un anhydride d'aminoacide de formule V 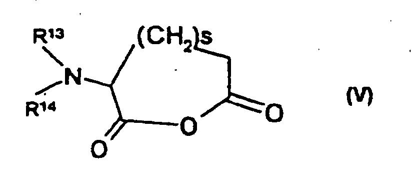 dans laquelle R¹³ représente un atome d'hydrogène et R¹⁴ un groupe protecteur de N connu dans la chimie des peptides, ou R¹³ et R¹⁴ forment un groupe protecteur de N cyclique, par exemple le groupe phtalimido, et s a la même définition que ci-dessus dans le procédé a),
avec un reste -N(R⁶)-R⁷ primaire ou secondaire, au sens d'une ouverture de cycle, pour obtenir un produit intermédiaire de formule VI 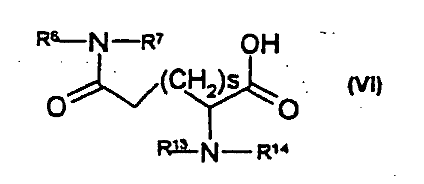 dans laquelle R¹³, R¹⁴, s, R⁶ et R⁷ ont la signification indiquée ci-dessus, cette ouverture de cycle donnant, en fonction du groupe protecteur et des conditions réactionnelles, de manière généralement régiosélective l'isomère représenté par la formule VI que, dans le cas de la formation de mélanges de régioisomères, on peut encore enrichir par cristallisation ou chromatographie, puis on sépare le groupe protecteur R¹³ et/ou R¹⁴ introduit en libérant l'amine, et on effectue ensuite, de la manière décrite dans a), une N-sulfonation avec un dérivé d'acide sulfonique de formule II, qui conduit à un produit de formule I, ou
c) on sépare en les énantiomères purs un composé de formule I préparé par le procédé a) ou b) qui, du fait de sa structure chimique, apparaît sous des formes énantiomères, par formation de sels avec des acides ou des bases énantiomères purs, chromatographie sur des phases stationnaires chirales ou formation de dérivés à l'aide de composés chiraux énantiomères purs comme des aminoacides, séparation des diastéréoisomères ainsi obtenus, et séparation des groupes auxiliaires chiraux dans les énantiomères purs, ou
d) on isole le composé de formule I préparé par les procédés a), b) ou c) sous forme libre, ou, dans le cas de la présence de groupes acides ou basiques, on le transforme en des sels physiologiquement acceptables.

7. Médicament **caractérisé par** une teneur efficace en un composé de formule I selon l'une ou plusieurs des revendications 1 à 5 avec un support ou un additif pharmaceutiquement approprié et physiologiquement acceptable et/ou d'autres substances actives et substances auxiliaires.

8. Utilisation d'au moins un composé de formule I selon l'une ou plusieurs des revendications 1 à 5 pour la préparation de médicaments destinés à la prophylaxie et au traitement de maladies dans l'évolution desquelles interviennent des métalloprotéinases matricielles.

9. Utilisation d'au moins un composé de formule I selon l'une ou plusieurs des revendications 1 à 5 pour la préparation de médicaments destinés à la prophylaxie et au traitement de maladies articulaires dégénératives comme les arthroses, la polyarthrite rhumatoïde, les spondyloses, des lésions du cartilage après un traumatisme articulaire ou une immobilisation prolongée de l'articulation, après des lésions du ménisque ou de la rotule ou des déchirures de tendon, ou de maladies du tissu conjonctif comme des collagénoses, des maladies parodontales, des troubles de la cicatrisation et des maladies chroniques de l'appareil moteur comme des arthrites inflammatoires, immunologiques ou métaboliques, aiguës et chroniques, des arthropathies, des myalgies et des troubles du métabolisme osseux comme l'ostéoporose, ou de maladies vasculaires, par exemple des occlusions de vaisseaux sanguins, des plaques d'athérosclérose ou des anévrismes, dans le cas de ruptures menaçantes ou de sténoses de pathogenèse quelconque, ou pour le traitement d'inflammations, y compris de plaies et d'ulcères, en particulier de la peau, de maladies cancéreuses, pour bloquer et circonscrire la formation de métastases et la propagation de métastases, dans le cancer du sein ou dans la cachexie, l'anorexie, le choc septique, la parodontose et la parodontite.

10. Procédé de préparation d'un médicament, **caractérisé en ce que** l'on met au moins un composé de formule I selon l'une ou plusieurs des revendications 1 à 5 sous une forme d'administration appropriée avec un support pharmaceutiquement approprié et physiologiquement acceptable et éventuellement d'autres substances actives, additifs ou substances auxiliaires.
